# EUROPEAN PATENT APPLICATION

(11) **EP 4 723 125 A1**
(43) Date of publication of application: **08.04.2026**
(21) Application number: 24815474.2
(22) Date of filing: 28.05.2024
(51) Int. Cl.: G16H 30/40

(54) **PROGRAM, ASSISTANCE METHOD, AND SYSTEM**

(30) Priority: 31.05.2023 JP 2023090400
(71) Applicant: Denka Company Limited, Tokyo 103-8338 (JP)
(72) Inventor: KOBUNA, Hiroyuki, Tokyo 103-8338 (JP); FUKUDA, Koichi, Tokyo 103-8338 (JP); ITAKURA, Satoshi, Tokyo 103-8338 (JP); OKUDA, Shujiro, Niigata-shi, Niigata 950-0917 (JP)
(74) Representative: Müller-Boré & Partner Patentanwälte PartG mbB
(86) International application number: PCT/JP2024/019507
(87) International publication number: WO 2024/247991

(57) **Abstract**

A program, an assistance method, and a system configured to be able to present useful information when performing an annotation are provided. A program for assisting in annotation of a medical image is provided. The program causes a computer to execute a presentation process for generating a presentation screen, the presentation screen presents to a user classification content of at least one label associated with a region image, which is an image of at least one region in the medical image, by at least one of a plurality of annotations.

## Description

### Technical Field

The present invention relates to a program, an assistance method, and a system for assisting in annotation of a medical image.

### Background Art

A data generation device in Patent Literature 1 generates training data for a machine learning algorithm. The training data is composed of a plurality of images and labels associated with each image. The term "annotation," depending on the context, refers to an act of annotating by labeling an image itself or a designated region within the image, or refers to a group of assigned labels.

### Citation List

### Patent Literature

[Patent Literature 1] International Publication No. 2021/206053

### Summary of Invention

### Technical Problem

Whether or not a medical image contains features such as a pathological abnormality can be a matter of differing judgment even among doctors with expert knowledge. Due to this, when performing an annotation of a medical image, a labeling judgments may differ when there are a plurality of entities performing the annotation, or when the same entity performs the annotation multiple times. The inventor has realized that the agreement and/or disagreement of content between a plurality of annotations can be a useful basis for judgment for an annotation worker.

The present invention provides a program, an assistance method, and a system configured to be able to present useful information when performing an annotation.

### Solution to Problem

According to the present disclosure, at least the following inventions are provided.
[1] A program for assisting in annotation of a medical image, wherein the program causes a computer to execute a presentation process of generating a presentation screen, and the presentation screen presents to a user classification content of at least one label associated with at least one region image in the medical image by at least one of a plurality of annotations.
[2] The program according to [1], wherein the plurality of annotations include a first annotation and a second annotation, the first annotation is performed by a first user, the second annotation is performed by a second user or is performed by the first user before the first annotation, and the presentation process causes the computer to generate the presentation screen so as to present to the first user the classification content of the at least one label associated with the at least one region image by the first and second annotations, when the first user is performing the first annotation or after completion of the first annotation.
[3] The program according to [1], wherein the plurality of annotations include a second annotation and a third annotation, when a first annotation is performed by a first user, the second annotation is performed by a second user or is performed by the first user before the first annotation, the third annotation is performed by a third user, is performed by the first user before the first annotation, or is performed by the second user before the second annotation, and the presentation process causes the computer to generate the presentation screen so as to present to the first user the classification content of the at least one label associated with the at least one region image by the second and third annotations, when the first user is performing the first annotation or after completion of the first annotation.
[4] The program according to any one of [1] to [3], wherein the at least one region image is defined for the medical image by a grid or by a designated frame in each annotation.
[5] The program according to any one of [1] to [4], wherein the at least one region image includes a label-comparable region image, the label-comparable region image is a region image with which a plurality of labels are associated by the plurality of annotations, and the presentation process causes the computer to generate the presentation screen so as to represent the classification content of the plurality of labels associated with the label-comparable region image by the plurality of annotations in a manner that allows a user to visually compare them.
[6] The program according to any one of [1] to [5], wherein the at least one region image includes a label-comparable region image, the label-comparable region image is a region image with which a plurality of labels are associated by the plurality of annotations, the program causes the computer to execute a process of calculating a comparison result from comparing the classification content of the plurality of labels, and the presentation screen presents the classification content of the plurality of labels associated with the label-comparable region image to a user by presenting the comparison result.
[7] The program according to any one of [1] to [6], wherein the presentation screen presents to a user the classification content of at least one label associated with a plurality of region images in the medical image by at least one of a plurality of annotations.
[8] The program according to any one of [1] to [7], wherein the presentation screen has a region image and a label image, the region image is an image of the at least one region in the medical image, the label image is an image corresponding to a label associated with the at least one region image, and the program causes a computer to execute a process of varying a display mode of the region image and/or the label image on the presentation screen according to the classification content and/or according to a comparison result of a plurality of the labels when the plurality of labels are associated with the region image.
[9] The program according to any one of [1] to [8], wherein the classification content of the label includes an index value or is an index value, and the index value represents how probable the classification content of the label is.
[10] An assistance method in annotation of a medical image, causing a computer to execute a presentation process of generating a presentation screen, wherein the presentation screen presents to a user classification content of at least one label associated with at least one region image in the medical image by at least one of a plurality of annotations.
[11] A system for assisting in annotation of a medical image, comprising a presentation unit that generates a presentation screen, wherein the presentation screen presents to a user classification content of at least one label associated with at least one region image in the medical image by at least one of a plurality of annotations.

According to the present invention, it is possible to present useful information when performing an annotation, by means of a presentation screen.

### Brief Description of Drawings

FIG. 1 is a diagram illustrating an example of a system 1 (a server 1), each user terminal 3, and a diagnostic device 4 according to an embodiment.
FIG. 2A is a block diagram illustrating an example of a hardware configuration of the server 1. FIG. 2B is a block diagram illustrating an example of a hardware configuration of the user terminal 3.
FIG. 3A is a block diagram illustrating a functional configuration of the server 1. FIG. 3B is a functional block diagram of a presentation unit.
FIG. 4 is a control flow representing a control operation of the embodiment.
FIG. 5 illustrates an example of a work screen 26 for performing an annotation of a grid method.
FIG. 6 illustrates an example of a scene where an input on a presentation screen 27 is accepted to issue a presentation instruction SN1 on the work screen 26 in the case of an annotation of a grid method.
FIG. 7 illustrates an example of the presentation screen 27 in a parallel presentation mode (number of calls: 1) and in a layer display, in the case of FIG. 6.
FIG. 8 illustrates an example of the presentation screen 27 in a parallel presentation mode (number of calls: 2) and in a layer display, in the case of FIG. 6.
FIG. 9 illustrates an example of the presentation screen 27 in a parallel presentation mode (number of calls: 2) and in a window display, in the case of FIG. 6.
FIG. 10 illustrates an example of the presentation screen 27 in a simple presentation mode (number of calls: 1) and in a window display, in the case of FIG. 6.
FIG. 11 illustrates an example of the presentation screen 27 in a simple presentation mode (number of calls: 2) and in a window display, in the case of FIG. 6.
FIG. 12 illustrates an example of the presentation screen 27 in a comparison presentation mode (number of calls: 2) and in a layer display, in the case of FIG. 6, comparing an annotation being worked on by a user with another single annotation.
FIG. 13 illustrates an example of the presentation screen 27 in a comparison presentation mode (number of calls: 2) and in a window display, in the case of FIG. 6.
FIG. 14 illustrates an example of the presentation screen 27 in a comparison presentation mode (number of calls: 2) and in a window display, in the case of FIG. 6, comparing the annotations of two other users (U001, U002) other than the logged-in user (U010).
FIG. 15 illustrates an example of the presentation screen 27 in a comparison presentation mode (number of calls: 2) and in a layer display, in the case of FIG. 6, comparing two other annotations from users other than the logged-in user.
FIG. 16 illustrates an example of the presentation screen 27 in a selection presentation mode (number of calls: 2, displaying only mismatched labels) and in a window display, in the case of FIG. 6.
FIG. 17 illustrates an example of the presentation screen 27 in a selection presentation mode (number of calls: 2, displaying only tumor labels) and in a layer display, in the case of FIG. 6.
FIG. 18 illustrates an example of the presentation screen 27 in a parallel presentation mode (number of calls: 2) and in a pop-up display, in the case of FIG. 6.
FIG. 19 illustrates an example of a scene where an input on a presentation screen 27 is accepted to issue a presentation instruction SN1 on a work screen 26 in the case of an annotation using an arbitrary designation frame method (a bounding box method).
FIG. 20 illustrates an example of the presentation screen 27 in a simple presentation mode (number of calls: 1) and in a layer display, in the case of FIG. 19.
FIG. 21A illustrates a part of the presentation screen 27 in a simple presentation mode (number of calls: 1) with grid setting ON and in a layer display, in the case of FIG. 19. FIG. 21B illustrates a parallel presentation mode (number of calls: 1) in the case of FIG. 21A. FIG. 21C illustrates a comparison presentation mode (number of calls: 1) in the case of FIG. 21A.
FIG. 22A illustrates a part of the presentation screen 27 in a parallel presentation mode with overlapping portion display (= grid setting OFF) and in a layer display, in the case of FIG. 19. FIG. 22B illustrates a comparison presentation mode (number of calls: 1) in the case of FIG. 22A. FIG. 22C illustrates an example of the presentation screen 27 with a number of calls of 2 in the parallel presentation mode of FIG. 22A.
FIG. 23A illustrates a part of the presentation screen 27 in a simple presentation mode (number of calls: 2) with grid setting ON and in a layer display, in the case of FIG. 19. FIG. 23B illustrates a parallel presentation mode (number of calls: 2) in the case of FIG. 23A. FIG. 23C illustrates a comparison presentation mode (number of calls: 2) in the case of FIG. 23A.
FIG. 24A is a diagram illustrating another example of a grid size. FIG. 24B is a diagram illustrating another example of an arbitrary designation frame (a bounding box).
FIG. 25A shows image display variations for contrasting or comparing annotations between self and others. FIG. 25B shows image display variations for contrasting or comparing a plurality of other annotations. FIG. 25C shows additional image display variations. FIG. 25D shows an example of a label with an index value.

### Description of Embodiments

Hereinafter, some embodiments of the present invention will be described with reference to the drawings. Various features shown in the embodiments described below can be combined with each other. In addition, an invention is independently established for each feature.

### <1. Term, etc.>

### (1-1. Annotation)

The term "annotation" in the field of machine learning, depending on the context, refers to an act of annotating by labeling an image, or refers to a group of assigned labels. An annotation as an act of annotating means "an act of associating annotation information as ground truth data with target information (electronic information) for machine learning." Various data such as images, videos, and audio can be targets for the annotation, and for example, an image annotation includes object detection, region extraction, and image classification. The image annotation associates a label with an annotation target. The annotation target may be each image (the entirety of the image) or an image of a region (an area) within the image. A label is also referred to as a tag or metadata. The label represents classification content (a classification result) and becomes the ground truth data in supervised learning. In an embodiment, "an annotation", unless a different definition is explicitly stated or a clear contradiction arises in the context, shall refer to "associating a label with a region image, which is an image of a region, when at least one region is designated in an image."

### (1-2. Annotation Target Image)

An image to be annotated in an embodiment shall include at least "a medical image". The medical image includes "an image that visualizes the structure or function inside the human body as an image, mainly for the diagnosis and treatment of diseases." The medical image may specifically include simple X-ray photography, computed tomography (CT), magnetic resonance imaging (MRI), ultrasound tomography (US), nuclear medicine examination, angiography, and the like. The medical image according to the present disclosure may further include "an endoscopic image" and "a pathological image" in addition to the various images described above. The endoscopic image may include, for example, images of the esophagus, stomach, intestines, and other digestive systems. The pathological image may specifically be "a (microscopic) image obtained by enlarging and photographing a specimen of tissue collected by an endoscope, surgery, or the like, which has been (1) fixed to prevent deterioration, (2) stained for easy observation, and (3) thinly sectioned to a few microns." The present invention is similarly applicable to any medical image, and any label classification type and classification content can be set. In the embodiment, a pathological image is adopted as an example of a medical image, the classification type of the label is a classification of whether it is a tumor, and the classification content of the label is "tumor" or "non-tumor."

### (1-3. Region and Region Image)

There are various methods for designating a region (an area) in a medical image. In the embodiment, a grid method is adopted in some examples, and a designated frame method is adopted in some other examples.

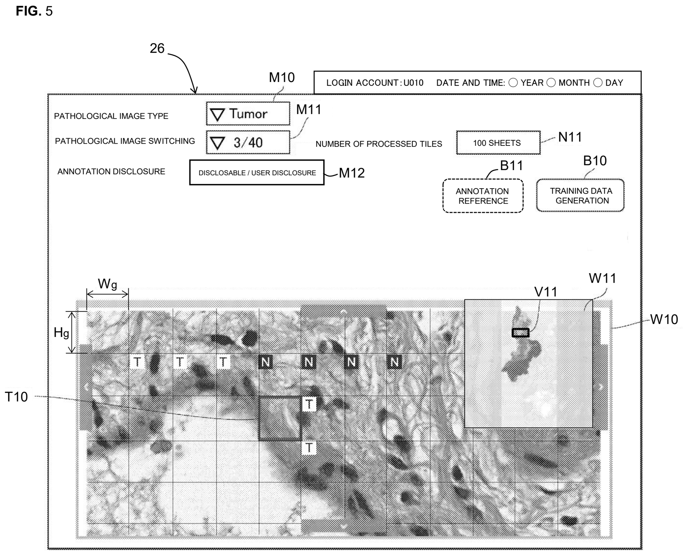

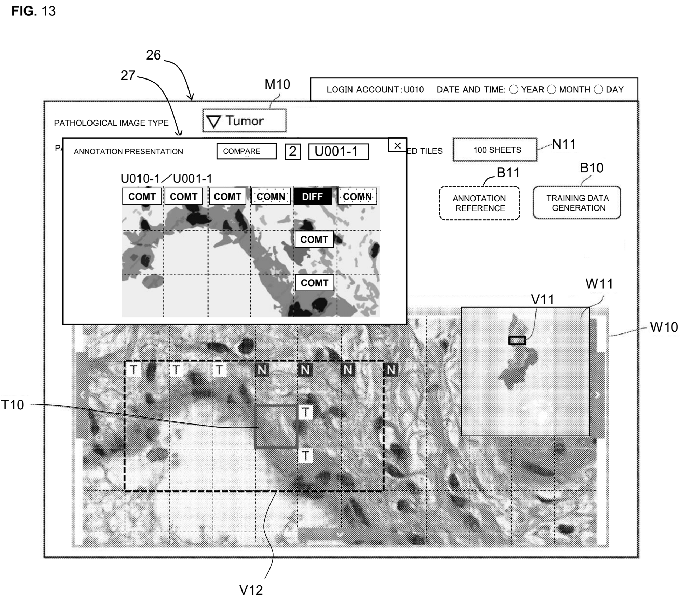

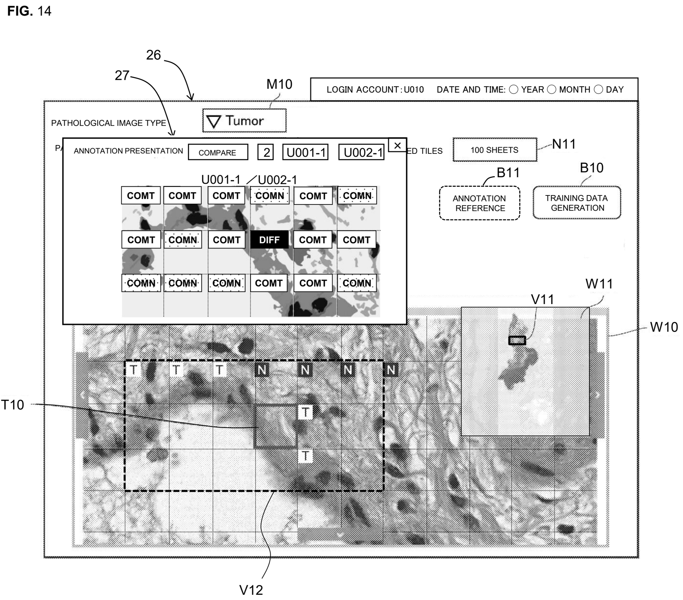

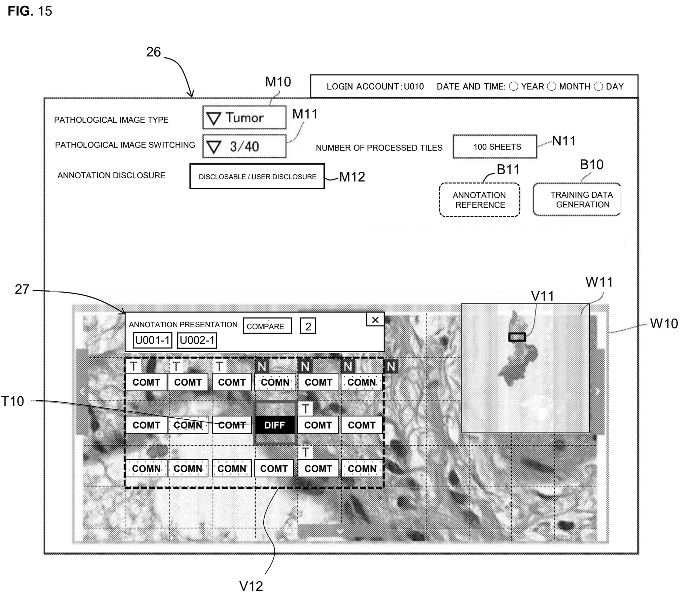

The "grid method" designates a region at a desired position and range in an image by means of a grid (i.e., a grid-like boundary line). When the grid has a plurality of cells, one of the cells of the grid can define one region. The grid may have uniform intervals, each region may be designated as a rectangle of a uniform size, and such a region may be referred to as a "tile." A grid interval is either defined or can be arbitrarily designated by a user. The "grid" mentioned here has a broad meaning and is not limited to a rectangular grid; a grid in which any tessellatable polygon is periodically arranged can be adopted. The grid may be, for example, a honeycomb grid or the like. The "honeycomb grid" here has a broad meaning, and may be, for example, a regular hexagonal honeycomb, but may also be, for example, a honeycomb structure of equilateral triangles or parallelograms, or a honeycomb structure of parallelohexagons (a hexagon in which three pairs of opposite sides are parallel but the side lengths differ between each pair). A region designated by the grid may be referred to as a "grid region" for convenience. In the embodiment, an annotation of the grid method is adopted in the examples of FIGS. 5 to 18.

The "designated frame method" is a method in which a user designates a region at a desired position and range in an image using an arbitrary designated frame. The designated frame is sometimes referred to as a bounding box. The designated frame can be set to any size and any shape, and may be, for example, but is not limited to, a rectangle, and may be any polygon or a closed curve. A region designated by the designated frame may be referred to as a "designated frame region" for convenience. In the embodiment, an annotation of a designated frame method is adopted in the examples of FIGS. 19 to 24B.

When a region is designated in an image, the image corresponding to that region is also referred to as a "region image." When the region image is an image designated in a uniform rectangular size by the grid method, this region image may be referred to as a tile image. The size of the region and the region image (the number of pixels in the vertical and horizontal directions) may be the same size as training data that can be input to a learning model. When a plurality of designated frames are set in an image, if two designated frames completely match, the designated frame regions match each other, but if each designated frame is different, the designated frame regions are also different. When there is an overlap between designated frames, an overlapping region where a plurality of designated frame regions overlap occurs.

The program, method, and system of the embodiment can adopt one or both of the grid method and the designated frame method. When both are adopted, which method to use in the current annotation may be set by a user's setting operation and/or by an automatic computer recommendation process.

### (1-4. Training Data)

Usually, an annotation is performed to construct a training dataset. When annotating a medical image, the training dataset is composed of a "training data image," which is a cropped medical image, and a label associated with the training data image. The size of the training data image (the number of pixels in the vertical and horizontal directions) is set to a size that can be input to a learning model. The "training data image" may be one obtained by cropping the medical image along a grid, or one obtained by cropping a part of the medical image with a designated frame (a bounding box). The training data image may be one obtained by cropping the medical image according to the region image at the time of annotation, that is, the region image at the time of annotation and the training data image may match. However, the present invention is not limited to this, and the training data image may be different from the region image at the time of annotation. As an example, when a medical image is annotated with a designated frame, the training data image may be generated by cropping it with a grid (for example, a rectangular grid with uniform intervals). As another example, when an annotation is made with a honeycomb grid or the like, a training data image may be generated by cropping this with another grid (for example, a rectangular grid with uniform intervals).

It should be noted that, at the stage during an annotation work, cropping of a medical image is not necessary. For example, each region image of the medical image is displayed on a work screen 26 and a presentation screen 27, which will be described later, but at this display stage, cropping of the medical image is not necessary. Cropping of the medical image may be performed at a stage where the annotation is completed and an instruction to create a training dataset is subsequently issued.

### <2. Configuration of the Embodiment>

### (2-1. Overview of System 1 and Diagnostic Device 4)

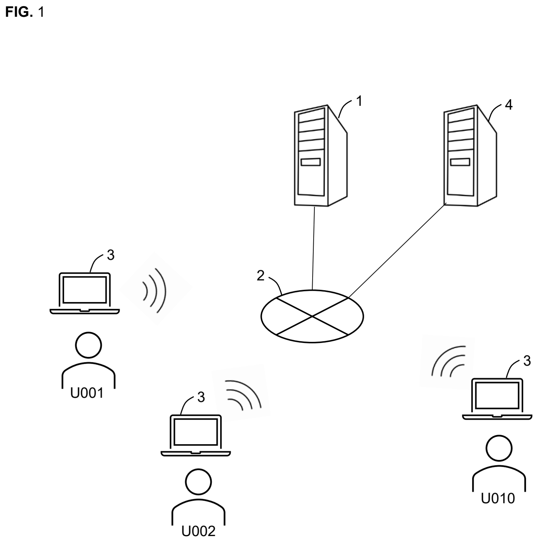

As shown in FIG. 1, a system 1, a user terminal 3, and a diagnostic device 4 of an embodiment are communicable with each other via a wireless or wired communication network 2. The system 1 in FIG. 1 can assist an annotation for a medical image. In the embodiment, the medical image is a pathological image as an example. The system 1 is a computer, specifically a server. The system 1 is provided by the server executing a program. For convenience, the system 1 may be referred to as a server 1. Users U001, U002, and U010 who use the system 1 are, for example, pathologists.

The system 1 includes a training data generation function (a training data generation unit). In the embodiment, the training data generation unit can generate training data using an input image (specifically, a medical image). The training data can be used as teacher data for training a learning model included in the diagnostic device 4. The diagnostic device 4 uses a trained model that has learned using the generated training data to determine whether or not features such as a pathological abnormality exist in a medical image.

The system 1 displays a medical image stored in the system 1 and a boundary line of a region image designated in the medical image superimposed on a screen. The system 1 accepts an annotation operation on the region image by a user. That is, the system 1 accepts an input of a label from the users U001, U002, and U010 for each region image displayed on the screen.

The system 1 may generate training data from a single medical image, or may generate training data from a plurality of medical images. When generating training data from a plurality of medical images, the system 1 repeats a process of designating one or more region images in a medical image and accepting a label input, for each medical image. When the user has completed assigning labels to all the medical images, the system 1 can generate training data. For training data generation, the system 1 first crops a medical image to generate a "training data image." The system 1 generates training data by associating image data of each training data image with a label assigned to each training data image (that is, a region image). The generated training data is sent to the diagnostic device 4.

The diagnostic device 4 trains the learning model using the training data sent from the system 1. A learning model is, for example, a neural network that can be given a predetermined ability through learning. The diagnostic device 4 inputs a medical image to be diagnosed into a trained model generated by learning, and based on an output result from the trained model, determines whether an abnormality exists in the medical image.

The system 1 can provide a presentation screen 27 as an assistance function to assist an annotation. The assistance function will be described in detail later.

### (2-2. Hardware Configuration of System 1)

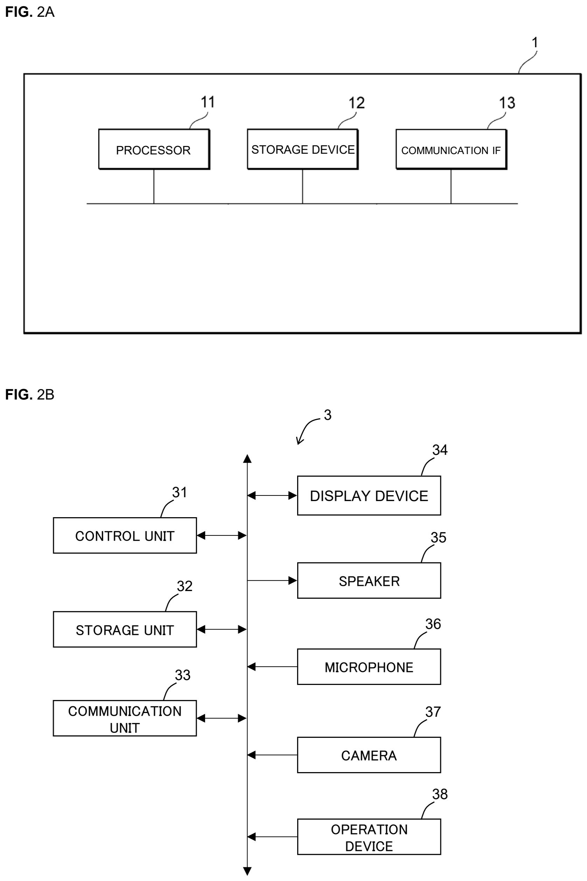

FIG. 2A is a diagram illustrating an example of a hardware configuration of the system 1. The system 1 includes a processor 11 such as a CPU (Central Processing Unit) or a GPU (Graphical Processing Unit), a memory, a storage device 12 such as an HDD (Hard Disk Drive) and/or an SSD (Solid State Drive), and a communication IF (Interface) 13 that performs wired or wireless communication. The system 1 accepts a user input operation from a user terminal 3 or causes a display device 34 (see FIG. 2B) of the user terminal 3 to display various screens via the communication IF 13.

### (2-3. Hardware Configuration of User Terminal 3)

As shown in FIG. 2B, a user terminal 3, as an example, includes a control unit 31 including a processor and the like, a storage unit 32 including a non-volatile memory and the like, a communication unit 33 for performing wireless communication and/or wired communication, a display device 34, a speaker 35, a microphone 36, a camera 37, and an operation device 38. The operation device 38 can adopt any input device such as a keyboard, a mouse, and/or a touch pen. The specific configuration of the user terminal 3 is not limited, and it may be various mobile terminals, smartphones, tablet terminals, or notebook PCs. When the user terminal 3 has a touch panel display, the display device 34 and the operation device 38 may be integrated into the touch panel display. The operation device 38 and the microphone 36 serve as an input interface (or input means) for a user to perform various input operations on the user terminal 3.

### (2-4. Functional Block Configuration)

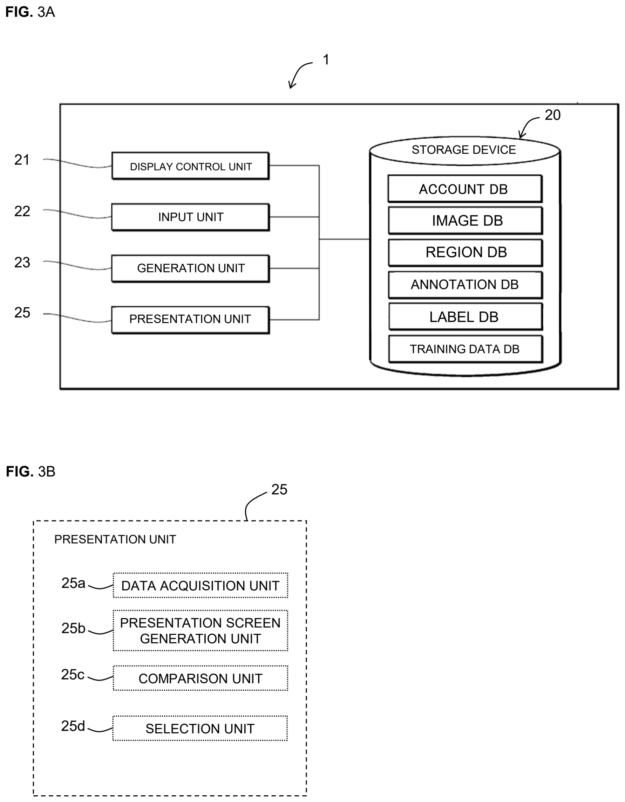

FIG. 3A is a diagram illustrating an example of a functional block configuration of the system 1. The system 1 includes a storage unit 20, a display control unit 21, an input unit 22, a generation unit 23, and a presentation unit 25. The storage unit 20 can be realized using a storage device 12 included in the system 1. The processor 11 of the system 1 executes a program stored in the storage device 12, thereby providing the display control unit 21, the input unit 22, the generation unit 23, and the presentation unit 25. The program may be provided over a network or stored in a storage medium. A storage medium storing the program may be a non-transitory computer-readable medium. The non-transitory storage medium is not particularly limited, but may be, for example, a storage medium such as a USB memory or a CD-ROM.

### (2-4-1. Configuration of Storage Unit 20)

The storage unit 20 includes various databases (DBs). The storage unit 20, as an example, stores an account DB, an image DB, a region DB, an annotation DB, a label DB, and a training data DB. In each DB, an identification ID is assigned to the stored data, and mutually related data can be cross-referenced.

The account DB stores each user's user ID, user detail information, login history, and the like. The user detail information may include an email address and the like, may include a name and the like associated with an account (a nickname, an avatar icon, etc.), and/or may include more personal and specific information such as a name and an affiliation.

The image DB stores one or more medical images (for example, pathological images) used for generating training data. The region DB stores information for identifying a region image in each medical image. As an example, the region DB stores, together with a region image ID, a medical image ID of a setting destination of the region image, and image coordinate data. The image coordinate data defines what range of the medical image each region image occupies.

The annotation DB stores annotation information for distinguishing each annotation performed using the system 1 from each other, together with an annotation ID. The annotation information is preferably information that can identify for which medical image, when, by whom, and what type of annotation was performed. The annotation information can preferably store "annotation entity information." The annotation entity information is identification information for identifying "who performed the annotation (i.e., the annotation entity)." The annotation entity information may be, for example, a user ID (for example, a login account), or may be any one or more pieces of information in the user detail information stored in the account DB. The annotation information may include disclosure availability of the annotation (i.e., whether it may be disclosed on a presentation screen 27 of another person with a different user ID), and may include a disclosure condition of the annotation (i.e., under what conditions it is disclosed to others).

The label DB stores information of a label associated with a region image by each annotation, in association with an annotation ID. The label DB stores each label ID and label detail information in association with each other. The label detail information includes a classification type and classification content of the label (for example, the type is tumor classification, and the label classification content is tumor, etc.).

The training data DB stores generated training data. A user can download any training data by referring to the training data DB.

It is preferable that at least the image DB, the region image ID, the annotation ID, and the label ID are associated with each other. This makes it possible to easily call up the content of any annotation (i.e., a medical image, a region image, a label, etc.) from the annotation DB by specifying the annotation ID. Note that the above data management and DB configuration are merely an example. Any data management and DB configuration can be adopted according to the specifications of actual hardware and/or software, etc.

### (2-4-2. Configuration of Other Functional Units)

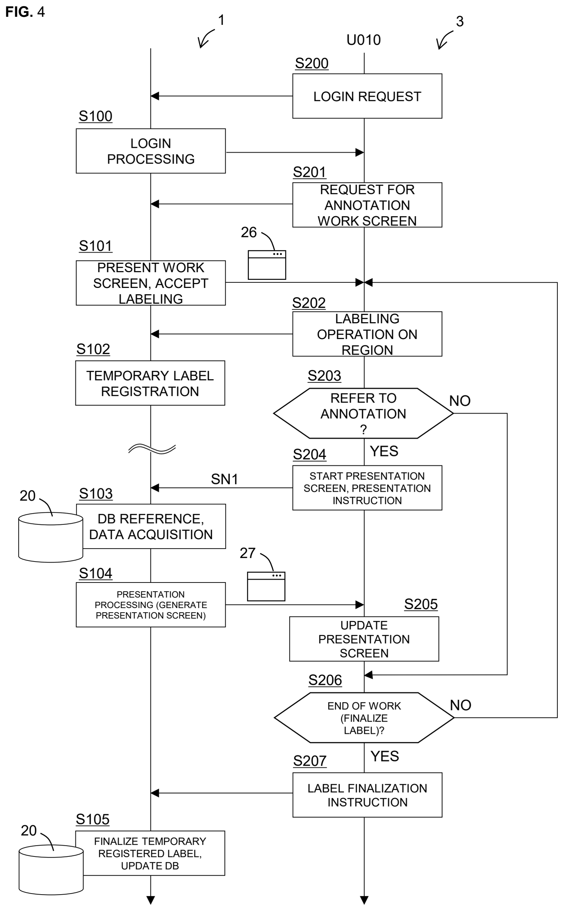

A display control unit 21 executes display control to cause a display device 34 of a user terminal 3 to display various screens. For example, the display control unit 21 causes a work screen 26 to be displayed, on which a boundary line of a region image is superimposed on a medical image. For example, the display control unit 21 performs control to provide a presentation screen 27 (see FIGS. 4, 6 to 18) generated by a presentation unit 25 (see FIG. 3B) either together with the work screen 26 or independently of the work screen 26.

An input unit 22 accepts various inputs from an operation device 38 of the user terminal 3. The input unit 22 accepts an annotation (a label input) from the operation device 38 of the user terminal 3. The label input is an input operation of which label to associate with each of a plurality of region images. The input unit 22 stores a label associated with each region image in a label DB. The input unit 22 may, for example, store a region image ID that uniquely identifies each region image and a label associated with each region image in the label DB in association with each other. The input of the label may be performed, for example, by selecting a region image for which to input a label from each region image displayed on a screen, and accepting a label designation to be associated with the region image.

As an example, a generation unit 23 generates training data for training a learning model by associating each of a plurality of region images with a label associated with each of the plurality of region images. For example, the generation unit 23 acquires a region image ID and a label from a label DB, and extracts image data of image coordinates corresponding to the region image ID from a medical image stored in an image DB. This extracted image data is a region image. The generation unit 23 can generate training data by combining the image data of the extracted region image and the label stored in association with the region image ID.

FIG. 3B is a functional block configuration of a presentation unit 25. The presentation unit 25 includes a data acquisition unit 25a, a presentation screen generation unit 25b, a comparison unit 25c, and a selection unit 25d. The data acquisition unit 25a can acquire information necessary for generating a presentation screen 27 (see FIGS. 4 to 18) from a storage unit 20. The presentation screen generation unit 25b can generate the presentation screen 27 based on the information acquired by the data acquisition unit 25a. The comparison unit 25c and the selection unit 25d, in a specific presentation mode (a comparison presentation mode, a selection presentation mode), calculate and/or select the information acquired by the data acquisition unit 25a, and can supply the information subjected to calculation, etc., to the presentation screen generation unit 25b.

### (2-5. Details of Work Screen 26)

Each of FIG. 5 and FIG. 19 is an example of a work screen 26 provided by the system 1. FIG. 5 is a work screen for an annotation of a grid method, and FIG. 19 is a work screen for an annotation of a designated frame method. The work screen 26 is a GUI for a user to perform an annotation work. The work screen 26 includes, as an example, selection menus M10, M11, and M12, a display area W10, an overview display area W11, a display frame V11, a display frame T10, a button B10 for generating training data, and a button B11 for referring to an annotation.

A selection menu M10 displays a type of a medical image (a pathological image as an example) being displayed. A selection menu M11 is a menu for designating a medical image (a pathological image as an example) to be displayed on the work screen 26 when a plurality of medical images are input. In the examples of FIG. 5 and FIG. 19, forty pathological images related to tumor cells have been input, and a third pathological image is currently displayed.

A selection menu M12 is a menu that allows a user who is performing an annotation work to select a disclosure setting (an access right) of the annotation to other users other than the user himself/herself. The selection menu M12 allows selection of at least either disclosable or non-disclosable. In the case of non-disclosable, only the user who performed the annotation can use that annotation. Use includes, for example, one or more of calling, referencing, duplicating, editing, and creating training data. In the case of disclosable, other users other than the user who performed the annotation can also use that annotation. In the case of disclosable, a disclosure condition can be further selected. The disclosure condition allows selection between "user disclosure" and "user concealment." A selection result of the selection menu M12 is registered in an annotation DB. In the case of user disclosure, when another user refers to an annotation via a presentation screen 27, annotation entity information of the user who performed that annotation is displayed on the presentation screen 27.

In an overview display area W11, an image of the entire medical image and a display frame V11 are displayed. The display frame V11 designates a region image to be enlarged and displayed in a display area W10 of the medical image. A user can arbitrarily change the position and size of the display frame V11.

In the example of FIG. 5, in the display area W10, a grid (a grid-like boundary line) is displayed superimposed on an enlarged image of a part of a medical image. In the example of FIG. 5, one cell of the grid corresponds to one "region image." In FIG. 5, as an example, one region image has a width W_{g} and a height H_{g}. Thus, in the embodiment, a computer (i.e., the system 1) is caused to execute a process of generating the display area W10 in which a grid (boundaries of a plurality of region images) is superimposed on a medical image. Each cell of the grid is stored in a region DB in association with position coordinates of each region image in the medical image. In the example of FIG. 5, a display frame T10 is displayed in the display area W10. The display frame T10 indicates a position of a region image for which a label input from a user is accepted. The position of the display frame T10 can be arbitrarily changed by an operation device 38. The display frame T10 can be placed at an arbitrary position, and a label can be input by an additional operation of the operation device 38.

In the example of FIG. 19, in the display area W10, several designated frames and labels are input on an enlarged image of a part of a medical image. One "region" can be designated by one designated frame. When the position and range of the designated frames differ between annotations, they are different regions from each other. In the embodiment, an operation device 38 includes a pointing device (a mouse as an example). The designated frame may be settable by a mouse operation as an example, and for example, a click position within the display area W10 may be set as a vertex of the designated frame. The designated frame is stored in a region DB in association with position coordinates of each region image in a medical image. As an example, by clicking on a designated frame, the designated frame becomes active (a selected state), and a label can be input by an additional operation of the operation device 38.

For a region image for which a label input has been completed, information indicating the classification content of the input label is displayed superimposed on the region image. In the embodiment, as an example, the label classification content is displayed with the characters "T" and "N". That is, when it is determined that a region image is an image of tumor cells, and as a result, a label indicating tumor cells is assigned to the region image, the character "T" is displayed at a predetermined position (the upper left as an example) for that region image. Also, when it is determined that a region image is not an image of tumor cells, and as a result, a label indicating that it is not a tumor cell is assigned to the region image, the character "N" is displayed at a predetermined position (the upper left as an example) for that region image.

A display control unit 21 may display an achievement value of the number of region images to which labels have been assigned in a display area image N11 of a work screen 26. This achievement value may be associated with each account and saved in a user account DB of a storage unit 20. The storage unit 20 may, as an example, total the achievement values of all medical images for each user. The storage unit 20 may, as an example, save an achievement value in association with each medical image, thereby making it possible to visually recognize a difference in achievement values between users for the same medical image.

### <3. Processing Flow of the Embodiment>

### (3-1. First Example)

A first example of the processing flow of the embodiment will be described with reference to FIGS. 4 to 7. The first example is an annotation of a grid method. The server 1 executes a program constructed according to the control flow of FIG. 4, thereby making it possible to assist an annotation for a medical image and providing an annotation assistance method.

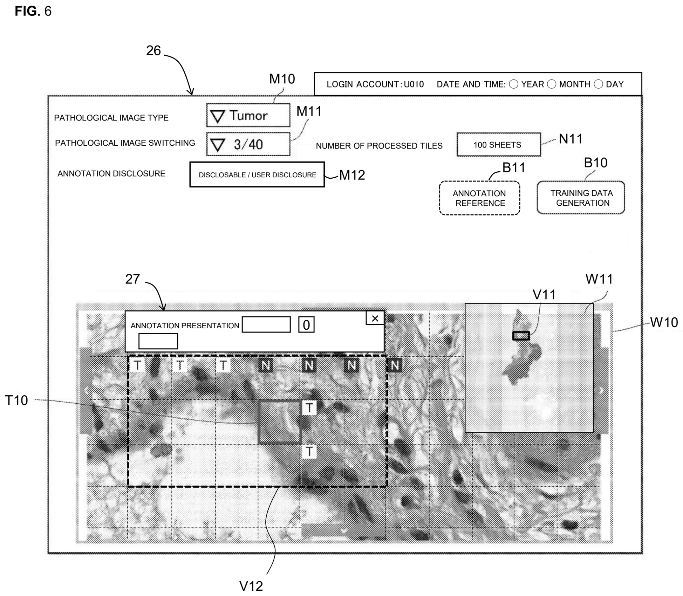

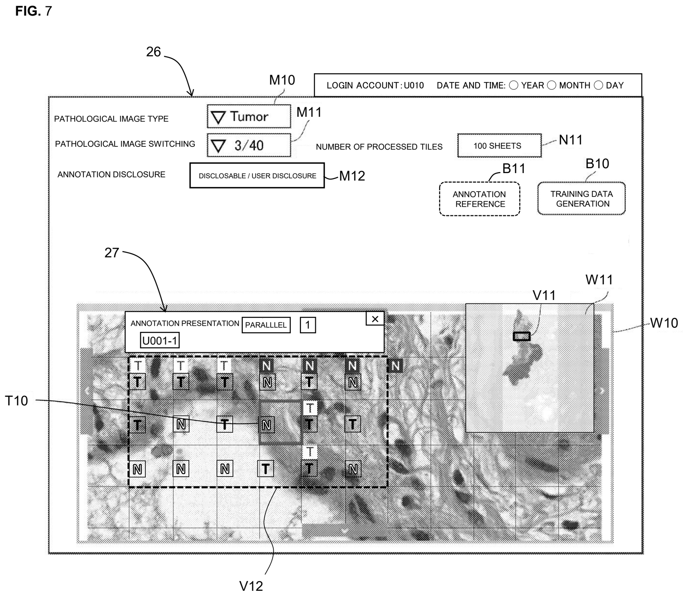

To give an overview of the flow in FIG. 4, after a login process (S200, S100), an annotation work (S201, S101, S202, S102) is performed using a work screen 26 of FIG. 5. According to a user's instruction, the system 1 presents a presentation screen 27 to the user (S203, S204, S103, S104, S205, FIGS. 5 and 6). By generating the presentation screen 27 illustrated in FIGS. 7 to 18, a user can refer to the classification content of at least one label associated with at least one region image in a medical image by a plurality of annotations.

Hereinafter, details of each step will be described. First, a user U010 logs into the system 1 in FIG. 4 (S200, S100). In the following description, the user U010 may be simply referred to as "the user."

After logging in, it is assumed that the user has requested a work screen for an annotation (S201). In this case, an input unit 22 accepts a selection of a medical image to be used for generating training data from the user. Note that the system 1 may set an access right to each medical image for each user account in an image DB. The user can select any medical image stored in the image DB, or can select only a medical image to which an access right has been granted according to a login account.

In step S101, the system 1, in response to an annotation work screen request from the user, outputs a work screen 26 (see FIG. 5) corresponding to the medical image and label classification type selected by the user. A display control unit 21 causes the work screen 26, on which a grid is superimposed, to be displayed on one medical image selected by the user. The grid is a boundary line of a region. The grid of the embodiment adopts, as an example, a rectangular grid having orthogonal vertical and horizontal axes, whereby a plurality of rectangular cells are defined, and one rectangular cell defines one region image. The system 1 enters a label assignment acceptance mode after outputting the work screen 26.

A label classification type is, for example, information regarding a type of pathology. As an example, the type of pathology may be either "tumor" or "hypermutation." For example, when each of a plurality of medical images is an image in which the presence of a tumor is suspected, "tumor" may be set as the type of pathology, and in that case, the classification content of a label can be "tumor" or "non-tumor." When each of a plurality of medical images is an image in which the presence of hypermutation is suspected, "hypermutation" may be set as the type of pathology, and in that case, the classification content of a label can be "hypermutation" or "non-hypermutation." The label classification type to be applied to a selected medical image may be automatically set by an input unit 22 according to a menu M10. Alternatively, the input unit 22 may accept a selection of a label classification type to be applied to the selected medical image from the user.

The user operates a user display frame T10 to input a label for each region image. In FIG. 5, the user U010 has finished inputting labels for some of the region images. The input unit 22 can accept a label input for each region image.

In step S202, a user terminal 3 transmits a user's label assignment operation from an operation device 38 to the system 1 each time. When a medical image is an image of a tumor, a label that a user assigns to each region image is either a label indicating that the region image is an image in which the presence of a tumor is suspected, or a label indicating that the region image is not an image in which the presence of a tumor is suspected. Also, when a medical image is an image of hypermutation, a label that a user assigns to each region image is either a label indicating that the region image is an image in which the presence of hypermutation is suspected, or a label indicating that the region image is not an image in which the presence of hypermutation is suspected.

In step S102, an input unit 22 associates a label with a region image ID based on label assignment operation information from a user terminal 3, and stores them as temporary registration information.

During an annotation work (S201 to S102), the user U010 may want to refer to another annotation. In this case, the user can click a button B11 for annotation reference on a user terminal 3.

In response to a click of a button B11, a determination result in step S203 becomes affirmative (YES), and a presentation screen 27 illustrated in FIG. 6 is activated. At this activation stage, label classification information has not yet been presented. At the same time, a presentation target frame V12 is displayed on a work screen 26. In the embodiment, as an example, a display control unit 21 displays the presentation target frame V12 in a display area W10. The presentation target frame V12 determines a target range of the presentation screen 27 (i.e., a region image for which a label by another annotation is presented). A user can select one or more regions over an arbitrary range within the display area W10 by operating an operation device 38, and can instruct the system 1 to set or update the presentation target frame V12.

By inputting necessary information into an input field of a presentation screen 27 in FIG. 6, a user can transmit a presentation instruction SN1 (see step S204 in FIG. 4) to the system 1. This presentation instruction SN1, as an example in the embodiment, includes a presentation range, a presentation mode, and call information. The "presentation range" can be set, for example, by a user arbitrarily selecting a region image using a presentation target frame V12 in FIG. 6. The "presentation mode," as an example, allows selection of one from simple presentation, parallel presentation, comparison presentation, and selection presentation. The "call information" specifies "which annotation to refer to" and "how many annotations to refer to," and specifically includes a "number of calls" and a "call annotation ID." The presentation screen 27 in FIG. 6 is provided with three input boxes (selection menus) for inputting the presentation mode, the number of calls, and the call annotation ID.

An example of a presentation instruction SN1 may be as follows. A region image within a presentation target frame V12 is a presentation target, the number of region images is 6 x 3 = 18 in total, a presentation mode is "parallel mode," a number of calls is "1," and a call annotation ID is "U001-1" (an example in which these are set is illustrated in FIG. 7). Note that the system 1 of the embodiment, as an example, allows a single user to perform an annotation on the same medical image multiple times. With an annotation ID "U001-1," a label from a first annotation by a user U001 can be called. By setting call annotation IDs as "U001-1," "U001-2," ..., first, second, ... annotations of the user U001 can be called. Note that the number of calls can be specified as two, or three or more arbitrary numbers. An example where the number of calls is two will be described later.

The system 1, in response to a presentation instruction SN1, starts a process for generating a presentation screen 27 (S103, S104).

First, a data acquisition unit 25a refers to a storage unit 20 according to a call annotation ID based on call information of a presentation instruction SN1. The data acquisition unit 25a refers to at least an image DB, a region DB, and a label DB of the storage unit 20, and thereby reads out a label of each region image to be presented on the presentation screen 27 (S103). The data acquisition unit 25a, as an example, refers to a region image ID of each region image within a presentation target frame V12 in order. The data acquisition unit 25a can extract all labels associated with one region image ID by searching the label DB with the region image ID as a search key. The data acquisition unit 25a compares an annotation ID of each extracted label with each call annotation ID of the presentation instruction SN1. The data acquisition unit 25a retains a label for which both accounts match as it is a presentation target, and excludes a label that does not match. Thereby, only the label to be presented can be extracted from the label DB.

In step S104, a presentation screen generation unit 25b generates a presentation screen 27 for displaying the label classification content of each region image, based on the information acquired by a data acquisition unit 25a. The presentation screen generation unit 25b transmits data for displaying the presentation screen 27 to a user terminal 3.

Upon receiving data for a presentation screen 27 from the system 1, a user terminal 3 updates the presentation screen 27 being displayed (S205). FIG. 7 is an example of the updated presentation screen 27. As can be seen by comparing FIG. 6 and FIG. 7, the presentation screen 27 in the example of FIG. 7 displays each label as a layer superimposed on each region image within a presentation target frame V12 of a display area W10. This allows a user U010 to compare a label during his/her own annotation work with a label from an annotation by another user U001. Note that for a region image that the user U010, who is performing an annotation work, has not yet labeled, only the label of the user U001 is displayed.

After that, the user U010 labels a predetermined number (or a predetermined range) for a medical image, and a timing to end the current annotation work arrives. In the embodiment, as an example, pressing a button B10 is considered the end of the work. When the user clicks the button B10, a work end process is performed (S206), a label finalization instruction is transmitted to the system 1 (S207), and the system 1 registers all labels that were in a temporary registration state in step S102 into a label DB (S105).

At the time of step S105, a generation unit 23 generates training data and saves it in a training data DB. As an example, a medical image may be cropped according to a grid of a work screen 26, whereby the size of a region image and the size of a training data image match. The training data is configured by each training data image and each label (see FIG. 5) that a logged-in user U010 associated with each region image on the work screen 26.

### (3-2. Second Example)

A second example of the processing flow of the embodiment will be described with reference to FIGS. 19 to 20. The second example is an annotation of a designated frame method. In the second example, as in the first example, a computer (the system 1) performs processing according to the processing flow of FIG. 4, but there are differences in steps S101, S202, and S104. In step S101, a work screen 26 of FIG. 19 is provided to a user U010. In step S202, the user U010 performs designated frame setting and label input for a medical image via the work screen 26 of FIG. 19. In the example of FIG. 19, annotation disclosure is enabled, and a disclosure condition is set to user concealment.

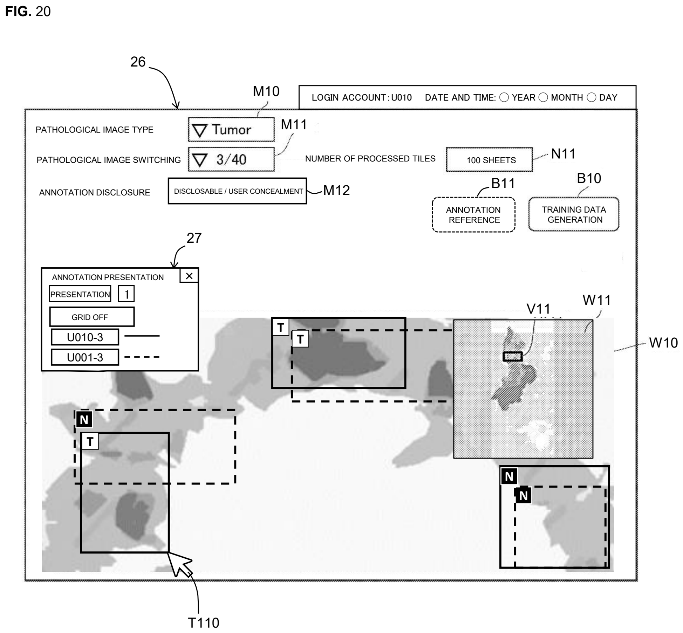

Also in the second example, similarly to the first example (FIG. 6), a user can activate a presentation screen 27 and input necessary information into an input field of the presentation screen 27, thereby enabling the user to transmit a presentation instruction SN1 (see step S204 in FIG. 4) to the system 1. However, in the second example, in step S104, a presentation screen 27 of FIG. 20 is presented to the user. As a point of difference from the first example (FIGS. 6 and 7), an annotation of the designated frame method (annotation ID = U010-3, U001-3) by a logged-in user (U010) and another user U001 is called.

As can be seen by comparing FIG. 19 and FIG. 20, the presentation screen 27 in the example of FIG. 20 displays several designated frames as a layer superimposed within a display area W10. Each designated frame is labeled, and a user can visually recognize the classification content of a label associated with a region image designated by each designated frame in a medical image.

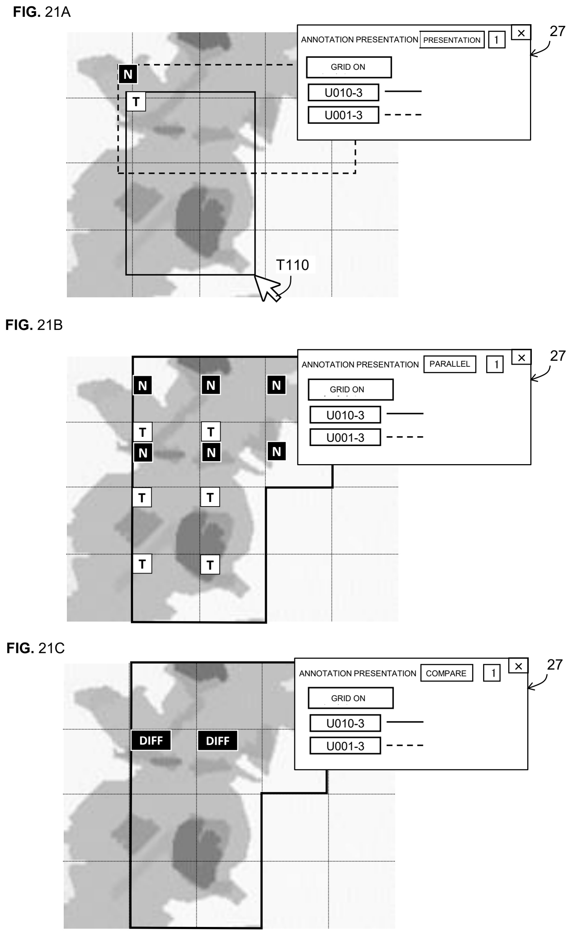

After that, similarly to the first example, when the process reaches step S105, a generation unit 23 generates training data and saves it in a training data DB. At this time, in the second example, there are variations in the method of cropping a medical image. As an example, a medical image may be cropped according to each designated frame on a work screen 26 of FIG. 19, and the cropped image may be used as a training data image. As another example, as illustrated in FIG. 21A, a grid may be further superimposed on a designated frame, and a region image within the designated frame may be cropped along the grid. In this way, also in the second example, training data can be configured.

### <4. Variations of Presentation Screen 27, Presentation Process, etc.>

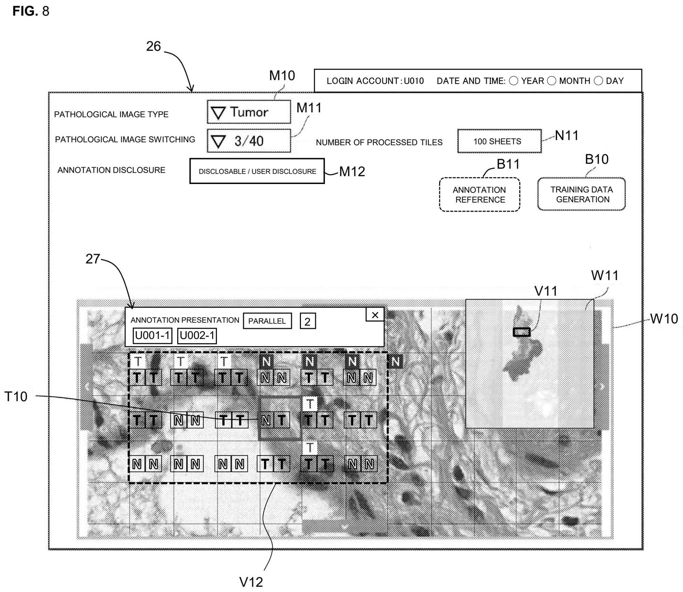

FIG. 7 (a grid method) and FIG. 20 (a designated frame method) are merely examples of a presentation screen 27, and at least FIGS. 8 to 18 and FIGS. 21 to 24B are further illustrated in addition to these. A presentation mode and a display method of the presentation screen 27 have various variations, and there are also various variations in the appearance of a region image and a label image. In addition, processes related to a presentation process also have various variations.

### (4-1. Variations of Presentation Mode)

The presentation mode of the embodiment can adopt at least one of a simple presentation mode, a parallel presentation mode, a comparison presentation mode, and a selection presentation mode. Among these, only one mode may be adopted, or a plurality of modes may be adopted so as to be switchable.

Examples of the "parallel presentation mode" are FIGS. 7 to 9, FIG. 18, FIG. 21B, FIG. 22A, FIG. 22C, and FIG. 23B. In this mode, a plurality of labels associated with a region image are arranged in parallel for each region. The direction of arrangement may be vertical, horizontal, or diagonal, and the labels may be partially overlapped.

The parallel presentation mode of FIG. 21B will be described. First, please refer to FIG. 21A. FIG. 21A is an enlarged view of the lower left region of FIG. 20. As an example, a region image conversion may be performed by further superimposing a grid on a designated frame.

This "region image conversion" refers to performing a conversion between a designated frame region and a grid region. For a region conversion, as an example, a label of a designated frame is associated with a region defined by a grid within a range covered by the designated frame. As an example, when an overlapping area of a designated frame region and a grid region is equal to or greater than a "predetermined overlap amount," the same label as that of the designated frame region image may be associated with the grid region image. The "predetermined overlap amount" is, for example, 20%, 30%, 40%, 50%, 60%, 70%, 80%, 90%, or 100% of the area of the grid region, and may be within a range between any two of the numerical values exemplified here. When the predetermined overlap amount is 100%, a certain grid region and a designated frame region completely overlap. In FIG. 21B, which is after the region image conversion, a group of regions with T labels in 3 rows and 2 columns and a group of regions with N labels in 2 rows and 3 columns partially overlap. After this, similarly to FIG. 7, labels can be arranged in parallel for each region image of the grid according to a predetermined rule. A display similar to that of FIG. 21B may be made over the entire display area W10 of FIG. 20.

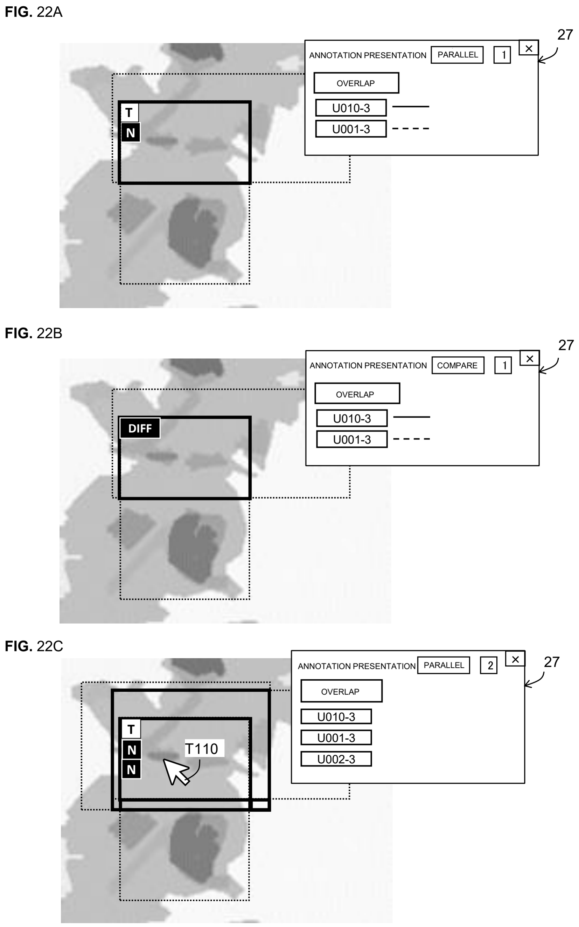

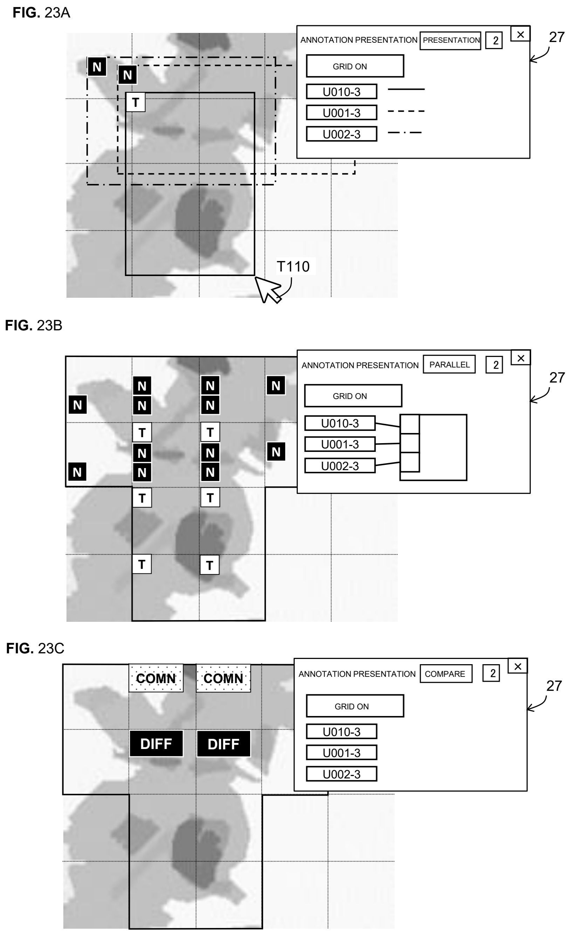

FIG. 22A, as an example, sets only an overlapping region where a plurality of designated frames overlap with each other as a presentation target region. FIG. 22C is similar, but as an example, three or more designated frames overlap. In FIG. 22C, as an example, a mouse pointer T110 position is set as an active region, and a parallel display of labels is performed only in the active region, which may improve visibility. FIG. 23B shows a situation where three designated frames of FIG. 23A overlap, and shows the state after a region image conversion has been applied to FIG. 23A.

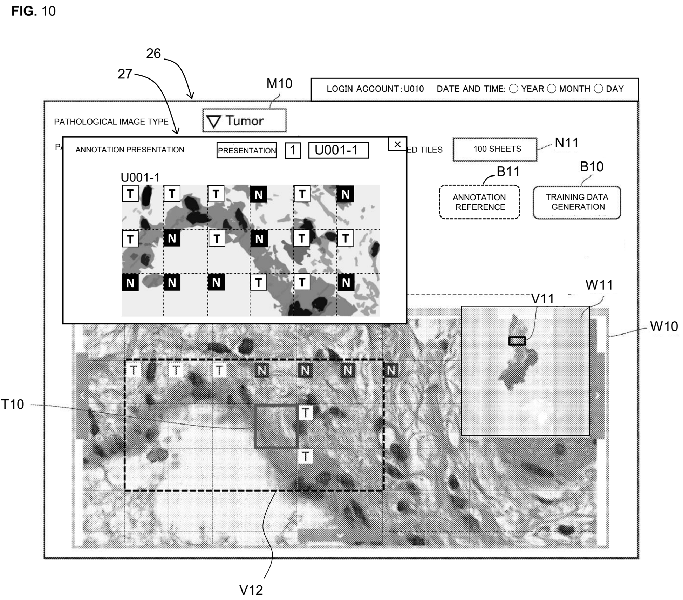

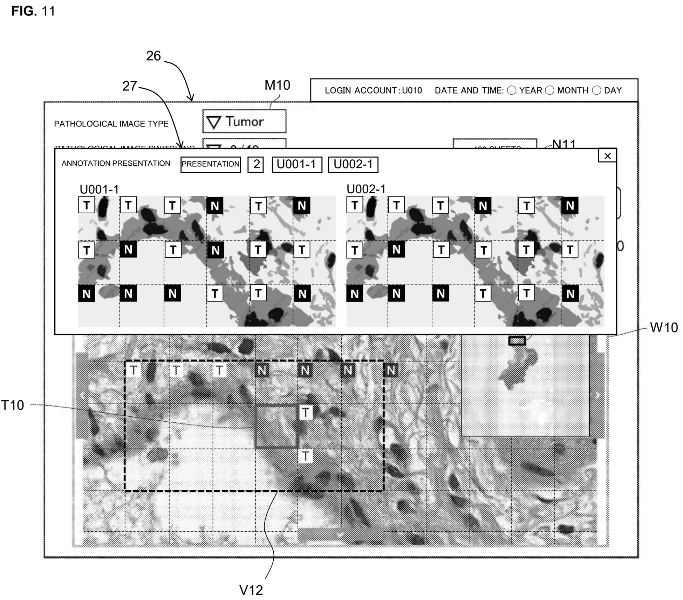

Examples of a "simple presentation mode" are FIGS. 10, 11, 20, 21A, 23A, and 24A-24B. In this mode, a set of a region group and a label group is generated for each annotation, and the set is arranged separately for each annotation. For example, in FIG. 10, a region image group within a presentation target frame V12 is displayed in a separate window on the upper side of a display area W10, and a label from an annotation by a second user U001 is displayed. In FIG. 11, two region image groups within the presentation target frame V12 are arranged side by side, and labels from each annotation by the second user U001 and a third user U002 are displayed.

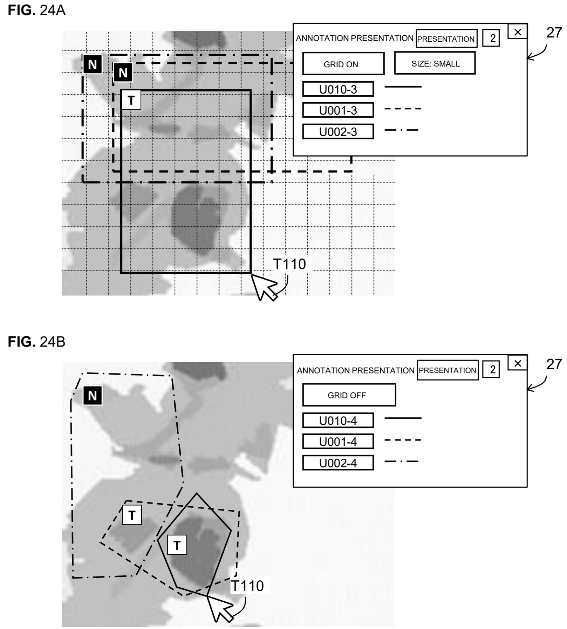

Similarly to FIG. 20, FIGS. 21A, 23A, and 24A to 24B are also in a simple presentation mode, but in FIGS. 21A, 23A, and 24A, a grid display is turned ON. Note that a grid size here is not limited, and may be set smaller as in FIG. 24A as an example, and may be arbitrarily adjustable by a user as an example.

FIG. 24B shows a designated frame other than a rectangle, and is a case of a polygonal designation frame as an example. Grid superimposition and region image conversion can also be applied to FIG. 24B. When a parallel presentation mode were to be adopted for FIG. 24B, only an overlapping region of a plurality of designated frames may be a presentation target, similarly to FIG. 22A and FIG. 22C.

In both a parallel presentation mode and a simple presentation mode, a plurality of labels associated with a label-comparable region image can be displayed in a comparable manner. A "label-comparable region image" is a region image with which a plurality of labels are associated by a plurality of annotations. In addition, a "label-comparable region image" is an image of a portion where a plurality of region images with which a plurality of labels are associated overlap, or the same region image with which a plurality of labels are associated, and it is possible to compare the associated plurality of labels. Like these modes, a computer may be caused to execute a presentation process of generating a presentation screen 27 so as to represent classification content of a plurality of labels associated with a label-comparable region image in a manner that allows a user to visually compare them.

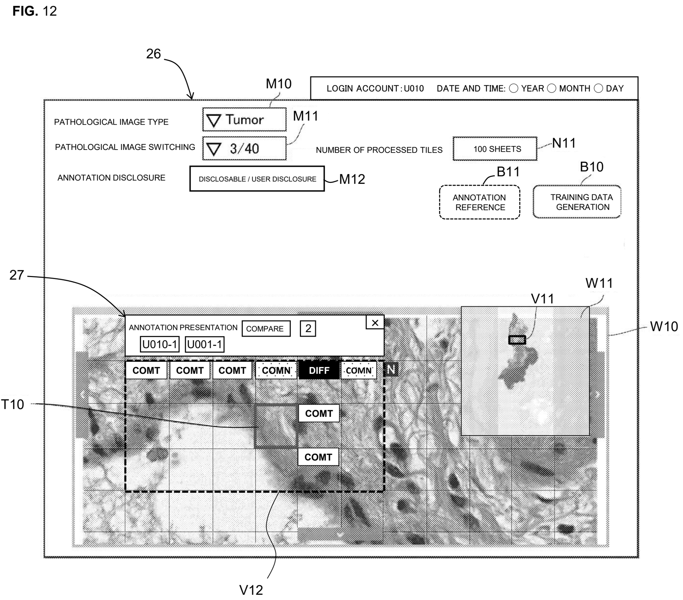

Examples of a "comparison presentation mode" are FIGS. 12 to 15, FIG. 21C, FIG. 22B, and FIG. 23C. The comparison presentation mode may cause a computer (a server 1) to execute a process (a process of a comparison unit 25c) of calculating a comparison result from comparing classification content of a plurality of labels associated with a label-comparable region image. A presentation screen 27 may present the classification content of a plurality of labels associated with a label-comparable region image to a user by presenting a comparison result. This comparison presentation mode presents classification content of a label to a user not by displaying the label itself, but by presenting a comparison result from comparing a plurality of labels. "Comparison" may be an agreement or a disagreement for same-type classification labels, and may be presence/absence of relevance determined according to a predetermined rule for different-type classification labels. When there are three or more comparison targets, in addition to unanimous agreement and disagreement, comparison results such as a majority in favor/a majority against/a balance may be shown. In FIGS. 12 to 15, FIG. 21C, FIG. 22B, and FIG. 23C, as an example, when labels match, "COMT" is displayed if all labels are "T," and "COMN" is displayed if all labels are "N," respectively. When labels do not match (disagree), "DIFF" is displayed.

There is no upper limit to the number of annotations that can be presented on a presentation screen 27, and it can be set arbitrarily. Therefore, there is also no upper limit to the number of labels to be displayed in a comparable manner in a parallel presentation mode or a simple presentation mode, and it can be set arbitrarily. Similarly, there is no upper limit to the number of labels to be compared with each other in a comparison presentation mode, and it can be set arbitrarily.

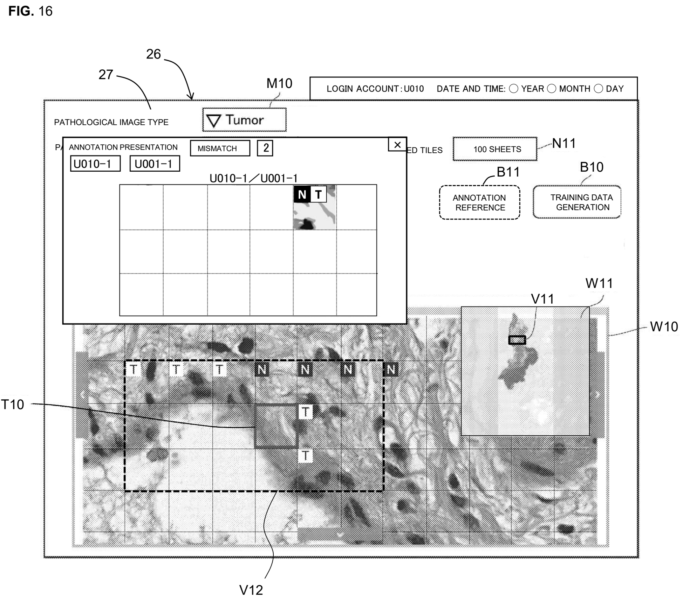

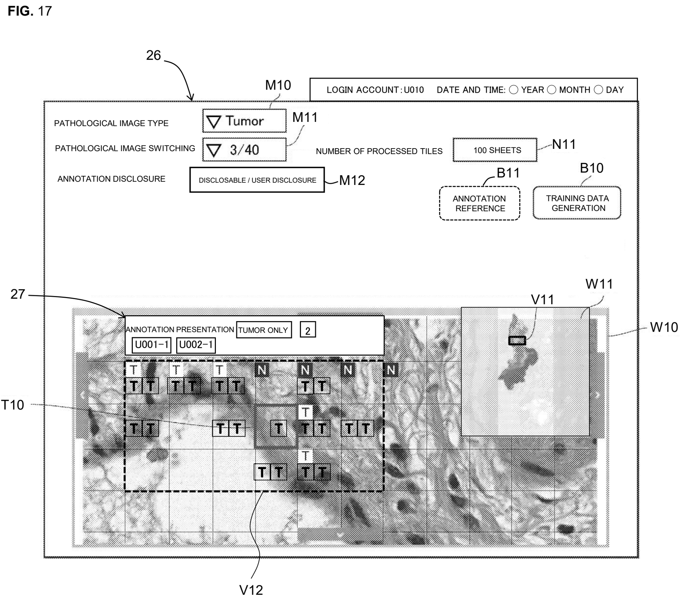

Examples of a "selection presentation mode" are FIGS. 16 and 17. In this mode, a selection unit 25d selects a presentation target label according to a selection item specified by a user, and then a presentation screen generation unit 25b generates a presentation screen 27. For example, a selection may be based on label classification content, and the presentation screen 27 may be generated by targeting only region images with mismatched labels (FIG. 16). A selection may be based on a label type. For example, the presentation screen 27 may be generated by targeting only region images with a tumor label ("T" label) from another annotation (FIG. 17). A selection presentation mode can also be adopted for an annotation of the designated frame method. For example, in FIGS. 22A, 22C, 23A, etc., only region images with mismatched labels may be presentation targets, or only designated frames with specific label classification content (for example, only T labels) or a specific label classification type (for example, only tumor/non-tumor labels) may be presentation targets.

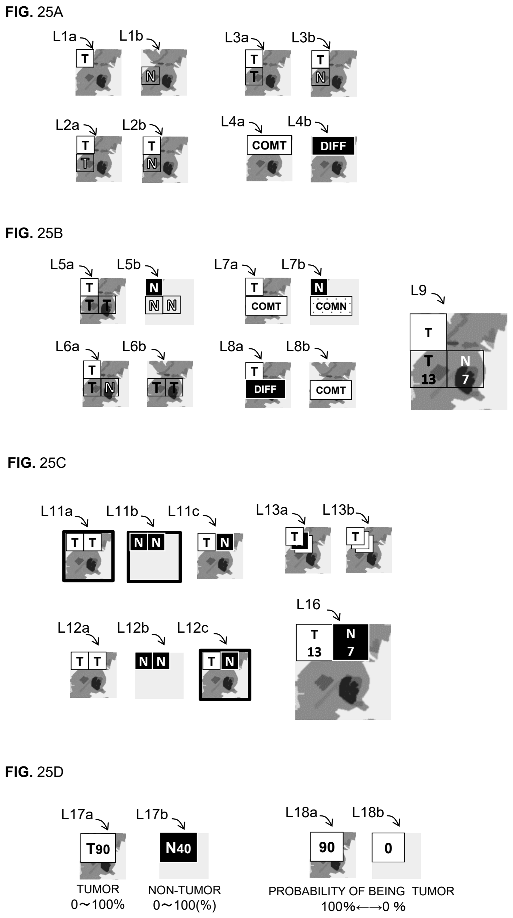

A plurality of presentation modes do not have to be selectable, and only one predetermined mode may be available, in which case a function for specifying a presentation mode can be omitted. Also, in a presentation instruction SN1, it may not be possible to selectively set call information (a call annotation ID). A setting function for call information may be omitted, all annotations for a presentation target medical image may be automatically called, or all labels associated with a presentation target region image may be automatically called. In this case, a specification of an annotation ID can be omitted. In these cases, since a large number of labels are usually associated with one region image, the number of assignments for each label content (for example, the number of assignments of tumor labels, the number of assignments of non-tumor labels) may be expressed numerically (see FIG. 25C).

### (4-2. Variations of Display Method)

The display method of the embodiment can adopt at least one of a layer display, a window display, and a pop-up display. Only one display method may be adopted, or a plurality of display methods may be adopted so as to be switchable. Any presentation mode can be combined with any display method.

Examples of a "layer display" are FIGS. 7, 8, 12, 15, 17, and 20 to 24B. As can be seen by comparing FIGS. 5 to 7, a presentation screen 27 is superimposed as a layer on a display area W10 of a work screen 26. That is, the presentation screen 27 displays labels for each region image superimposed on the display area W10. Individual label images may be transparent to make a region image easy to see, but they may also be opaque.

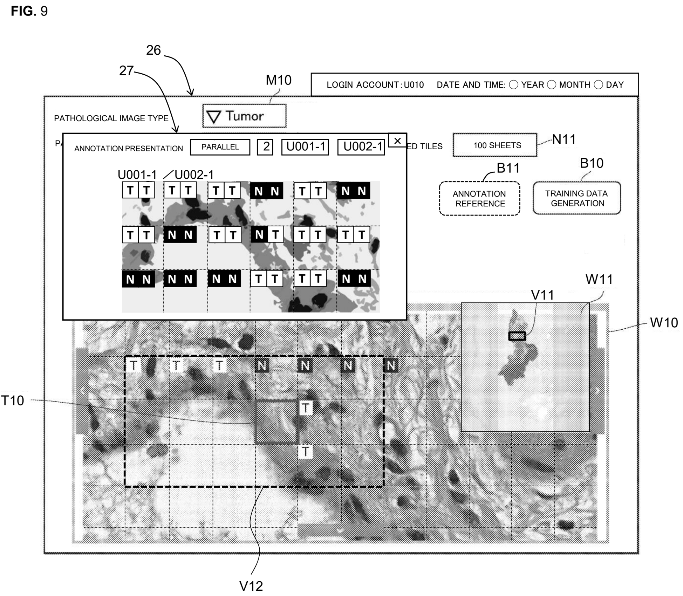

Examples of a "window display" are FIGS. 9 to 11, 13, 14, and 16. A presentation screen 27 is displayed as a separate window on a work screen 26. Note that in these drawings, while a display area W10 is a photograph, a region image of the presentation screen 27 is illustrated in an illustrative style, but this is merely for convenience of illustration and does not limit the present invention. The region image of the presentation screen 27 may be the same as the photograph of the display area W10. This point is also the same for a pop-up display described below. A window display can also be adopted for an annotation of the designated frame method, and a medical image, a designated frame, and a label of another annotation can be displayed in a separate window.

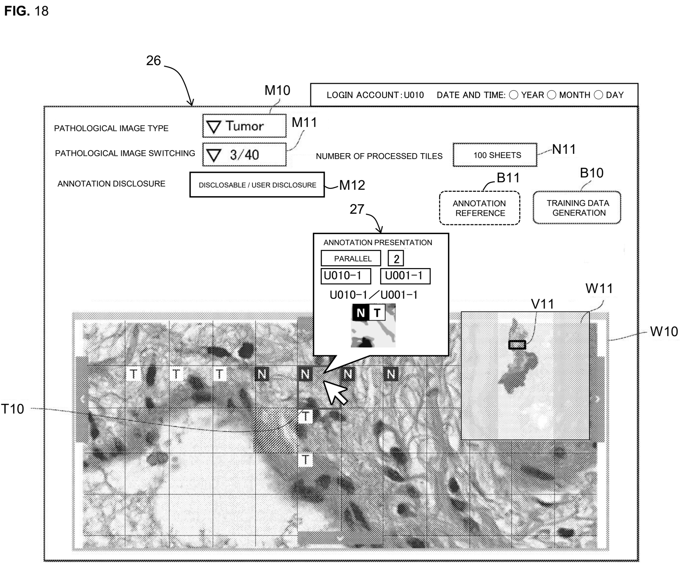

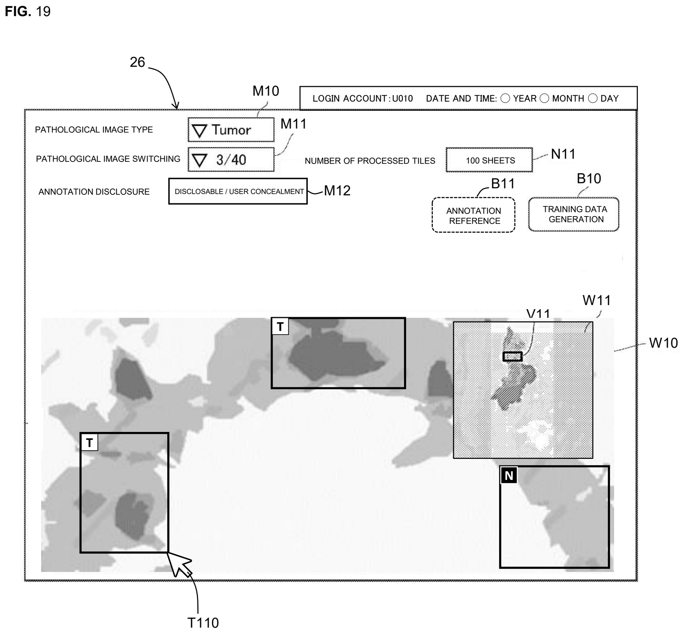

An example of a "pop-up display" is FIG. 18. Near a selected region image, the label classification content of only that region image is displayed in a pop-up window. As another example, a pop-up display may be adopted in FIG. 20, for example. For example, when a mouse pointer T110 is placed at the lower left position of a display area W10 as in FIG. 20, and a pop-up display command is issued near a designated frame, a display like that of FIG. 21B, FIG. 22A, or FIG. 22B may be displayed in a pop-up window.

### (4-3. Number of Presentable Annotations, Number of Region Images, and Number of Labels)

In an embodiment, a presentation screen 27 presents to a user classification content of at least one label associated with at least one region image by a plurality of annotations. The terms "at least one region image" and "at least one label" are described with the intention of including the following aspects. In the embodiment, an annotation method (a grid method or a designated frame method), each presentation mode, each display method, and other various modified examples can be adopted in any combination, and the number of region images to be presented on the presentation screen 27 can be variously changed according to the combination. Also, the number of labels changes depending on the presence or absence of a missing label. A missing label is caused by, for example, a missed label input in an annotation.

Specifically, for example, by a pop-up display of the embodiment (see FIG. 18), it is possible to present to a user the classification content of at least one label for a single region image. In FIG. 18, two labels are illustrated, but when there is a missing label in any of a plurality of annotations (U010-1, U001-1), the number of labels associated with one region image may be less than two. Thus, according to a presentation screen 27 that adopts a pop-up display (see FIG. 18), it is possible to present at least one piece of label classification information for a single region image. Also, when a presentation target frame V12 selects only one region image in a layer display or a window display, it is the same as a pop-up display. As another example, when two designated frames are provided at substantially the same position and range in FIG. 21A, one region image is being labeled by a plurality of annotations. As yet another example, each designated frame in FIG. 21A may be smaller than illustrated, and may be substantially as small as a grid size. In this case, a situation where two designated frames surround one region image by a grid, and a label is associated only with that region image may occur. In these cases as well, a missing label can similarly occur. Even in this situation, the presentation screen 27 can present at least one piece of label classification information for a single region image.

On the other hand, in some examples in the embodiment, a presentation screen 27 can present to a user classification content of a plurality of labels associated with a plurality of region images in a medical image by a plurality of annotations. For example, in the embodiment, the presentation screen 27 can be generated in a state where a plurality of region images are selected in a presentation target frame V12, or with the entire display area W10 including a plurality of region images as a target, or by a pop-up display targeting a plurality of region images. In this case, the classification content of "a plurality of labels" associated with "a plurality of region images" can be presented to the user.

Alternatively, for example, when no label is missing in a plurality of annotations, a plurality of labels are associated with all of the region images by the plurality of annotations. Also in this case, a presentation screen 27 can present to a user the classification content of the plurality of labels associated with the plurality of region images by the plurality of annotations.

Note that, as a modification, in, for example, step S104 or the like in the processing flow of FIG. 4, a computer (a system 1) may be caused to notify a user that a label is missing. For example, a label may be missing for a specific region image in some or all of the plurality of annotations. In that case, for example, in step S104 or the like, the computer (the system 1) may be caused to present a notification signal (an error message or the like) to the user instead of and/or together with the presentation screen 27, or the specific region image may be displayed in a highlighted manner.

A presentation target frame V12 may be omitted. In this case, as an example, all region images in a display area W10 may always be targeted. Also in this case, the presentation screen 27 can present to the user the classification content of the plurality of labels associated with the plurality of region images by the plurality of annotations.

Note that, in the embodiment, first to third annotations are illustrated for convenience. However, there is no upper limit to the number of annotations that can be presented on the presentation screen 27, and there is no upper limit to the number of labels that can be presented in association with one region image within the presentation screen 27. The system 1 can store an arbitrary number of annotations by an arbitrary number of users in a storage unit 20, and thereby an arbitrary number of labels are associated with one region image ID in a label DB. The system 1 may read out three or more arbitrary annotations (a set of a region image and a label) from an image DB and the label DB, and may thereby present the classification content of three or more arbitrary labels for each region image on the presentation screen 27.

Here, the maximum number of annotations that the system 1 can present on the presentation screen 27 is set to "Z". A value of Z is, for example, 1 to 100, for example, 2 to 20, and may be, for example, 3 to 10. The value of Z is specifically, for example, 1, 2, 3, 4, 5, 6, 7, 8, 9, 10, 15, 20, 30, 40, 50, 75, or 100, and may be within a range of any two of the numerical values exemplified here. When there is no missing label in each annotation, as the number of annotations increases, the number of labels associated with one region image also increases in proportion to this. Therefore, for example, when Z=5, a maximum of five labels can be associated with one region image within the presentation screen 27.

The system 1 of the embodiment may be used by limited members, for example, may be used only by members within a certain company, a laboratory, or a research group. However, the present invention is not limited to this, and the system 1 may be able to accept user account registration from members of many more various research institutions, universities, or companies, and may be accessible by users in any region of the world, and for that purpose, may be widely disclosed to the public through a communication network 2. This has an advantage that annotation data can be collected from many more various users. However, in that case, not all users are necessarily favorable to disclosing their own user information. In order to address this point, the system 1 of the embodiment includes a restriction function related to annotation disclosure and/or a later-described "display/non-display function of annotation subject information".

### (4-4. Addition of performance value, display/non-display of annotation subject information)

A "performance value" may be reflected on the presentation screen 27. The performance value is a cumulative number of labeling for each user. The "performance value" may be incorporated in any presentation mode. For example, a label associated by a user with a high performance value may be highlighted more than a label associated by a user with a low performance value. In a selective presentation mode, only labels of users whose performance value is a certain value or more may be selected and presented.

Annotation subject information may be displayed on the presentation screen 27. The annotation subject information is information that can identify who an annotation subject is that has input each label. For example, in FIG. 8 and the like, "U001-1" and "U002-1" are displayed in an input box for a call annotation ID, and this functions as the annotation subject information. For example, in FIG. 11, there are two sets of a region image and a label display region image in the presentation screen 27, but "U001-1" is added to one region image, and "U002-1" is added to the other region image, so that the annotation subject information can be easily distinguished. This is an example, and as another example of the annotation subject information, a user's name, an affiliation, a nickname, an avatar icon, or the like may be displayable on the presentation screen 27.

However, on the other hand, the annotation subject information may not be displayed. As an example, subject information of an annotation called to the presentation screen 27 may not be displayed. For example, when a call mode is an all call mode, the annotation subject information may not be displayed at all. An annotation set to "non-disclosable" in a selection menu M12 in FIG. 5 or the like may be made callable on condition that the annotation subject information is not displayed. By not displaying the annotation subject information, an annotation subject can be concealed. Even if a certain user is not favorable to disclosing their own user information, there is an advantage that the annotation data of that user can be utilized while ensuring anonymity. An example of utilizing the annotation data while ensuring the anonymity will be additionally described later in variations of the call mode.

### (4-5. Variations of display mode, label appearance, etc.)

There are various variations for a means for distinctively displaying the classification content of a label associated with "at least one region image in a medical image". For example, as shown in FIGS. 25A to 25D, there are various variations in the appearance of the label.

Images L1a to L4b illustrated in FIG. 25A can be used for contrast or comparison between an annotation of oneself and an annotation of another person, or a plurality of annotations of oneself. An image L1a is an example of a label display of oneself (a logged-in user), and an image L1b is an example of a label display of another person, and both can be displayed in parallel by shifting their positions vertically. Note that the same display mode may be adopted for a self-label and an other-person-label, and colors and the like need not be made different. In images L2a and L2b, other-person-labels have the same color, and in images L3a and L3b, other-person-labels have different colors (T is black, N is white). Images L4a and L4b are comparison labels (match/mismatch), and have different characters and different colors.

FIG. 25B illustrates an example of contrast or comparison of a plurality of annotations of other persons. As in images L5a to L6b, a plurality of other-person-labels may be displayed in parallel below the self-label. As in images L7a to L8b, a comparison result label may be displayed below the self-label, and COMT and COMN may be displayed in different colors. When it is desired to present three or more other-person-labels in a comparable manner, the classification content of the labels associated with the region image may be displayed so as to be visually distinguishable. For example, it may be expressed by a numerical value or the like as in an image L9, and in this example, the number of "T" labels is 13 and the number of "N" labels is 7.

In FIG. 25C, images L11a to L11c highlight only matching labels with a thick frame, and images L12a to L12c highlight only mismatching labels with a thick frame. An arbitrary number of labels may be partially overlapped and displayed as in images L13a and L13b. An image L16 is a modification of the image L9.

Generalizing or extending the above example, a "difference in display mode" may, for example, statically and/or dynamically make the appearance of a region image or a label image different. "Statically different" may, as an example, apply highlighting, a color difference, or the like to a specific region image or a specific label, or, as another example, superimpose an arbitrary additional image other than a label on a specific region image for display. The "additional image" may be an image representing an arbitrary symbol, a color, and/or a pattern. The "symbol" may be, for example, a character symbol, a mathematical symbol, or a graphic symbol. A shape difference, a color difference, and/or a pattern difference of the label may be applied. Highlighting is to make the region image or the label stand out by an arbitrary method such as a thick frame or an enhanced display. A color difference and/or a transparency difference may be adopted for the region image. The color difference may be, for example, color coding of monochrome/color, or color coding with an arbitrary plurality of colors. For the transparency difference, for example, one region image or label image may be displayed with transparency, while the remaining region image or label image is displayed non-transparently, so that a user can distinguish and visually recognize these region images. "Dynamically different" may, as an example, add an animation (a movement of a display) to a specific region image or a specific label image, and the animation may include blinking, a repeated change in transmittance, and/or a repeated change in size.

As described above, when the presentation screen 27 includes a region image and a label image, the display mode of the region image and/or the label image may be made different on the presentation screen 27. Specifically, a process (S104) of making the display mode of the region image and/or the label image different according to the classification content of the labels and/or according to a comparison result of a plurality of labels associated with one region image may be executed by a computer (the server 1).

### (4-6. Addition of index value)

As shown in FIG. 25D, the classification content of a label associated with a region image as an annotation target may include an index value, and for example, in images L17a and L17b, the index value may be displayed as a subscript for "T" and "N". The index value represents how probable the classification content of the label is. The index value may be, for example, manually input by a user during an annotation work on a work screen 26. The index value can be adopted in an annotation of either a grid method or a designated frame method. The classification content of the label may be the index value, and for example, in a display example of the images L17a and L17b, the closer a numerical value is to 100, the more the possibility of a tumor is 100%, and the closer the numerical value is to 0, the more the possibility of a tumor is 0%, that is, non-tumor. The index value may be arbitrarily set by the user with an arbitrary step width (for example, a constant width such as in 1% increments or 5% increments), or the index value may be presented as, for example, a plurality of options (for example, an arbitrary number of stages such as three stages or four stages), and a desired index value may be able to select by the user from them. For example, a three-stage index value may be "high" (for example, 70% or more), "medium" (for example, 31% to 69%), and "low" (for example, 30% or less). There is an advantage that the convenience of a user's index value input is improved. In the case of a comparison presentation mode, a calculation between index values can also be performed. As an example, a calculated value (for example, a total value, an average value, etc.) may be calculated for the index values of a plurality of "T" labels, and the calculated value may be written on the labels. Alternatively, a similarity between a plurality of labels may be calculated in the comparison presentation mode. As a method for displaying the similarity, for example, a difference in the similarity between the labels may be displayed on the labels as a numerical value, or a determination result determined based on whether a difference in the index value between the labels is equal to or less than a predetermined value may be displayed on the labels.

### (4-7. Variations of call mode)

The system 1 of the embodiment can be provided with various call modes. As an example, the call mode of the embodiment can adopt any one of an annotation designation call mode, an all call mode, and a stratified call mode. A user may be able to selectively use two or more of these modes. An arbitrary call mode can be adopted together with an annotation method (the grid method or the designated frame method) of the embodiment, each presentation mode, each display method, and other various modifications.

In the embodiment, the annotation designation call mode for inputting an annotation ID to the presentation screen 27 is mainly described (see, for example, FIG. 7 and FIG. 20). The all call mode is a mode in which all annotations associated with a medical image at the time of calling are automatically called and presented on the presentation screen 27.

The stratified call mode is a mode in which only an annotation corresponding to a specified condition is extracted and presented on the presentation screen 27. Various types of stratification can be set. For example, it may be a stratification based on whether the annotation subject information corresponds to a predetermined condition. For example, only an annotation whose performance value is a predetermined value or more may be extracted, and thereby only a highly-proven and reliable annotation can be presented. For example, a period may be specifiable as one of call conditions, and only an annotation that has been registered (specifically, newly registered or finally registered for editing) within a specified period may be extracted. As another example, the index value may be used, and for example, only an annotation in which a summary statistic of all labels in the annotation (for example, an average index value or a median value of index values of all labels) is a predetermined threshold value or more may be extracted, and thereby it becomes possible to extract only an annotation created by an annotation subject with a certain degree of confidence.

As an example, the system 1 of the embodiment is configured to be switchable between a case of a "disclosure mode" in which the annotation subject information is displayed on the presentation screen 27 and a case of a "non-disclosure mode" in which the annotation subject information is not displayed. In the case of the disclosure mode, an arbitrary call mode can be adopted. Note that, in the case of the disclosure mode, an annotation set to "disclosable/user concealment" in the selection menu M12 in FIG. 5 or the like may be prohibited from being called on an annotation DB.

On the other hand, in the case of the non-disclosure mode, there is a restriction on an operable call mode. In the non-disclosure mode, at least a mechanism in which the annotation subject information can be recognized, for example, the annotation designation call mode in which an annotation ID including a user ID is input, cannot be adopted. The all call mode and the stratified call mode can also be adopted in the non-disclosure mode, and in these call modes, the display of the annotation subject information on the presentation screen 27 or the like may be prohibited.

### <5. Features and Effects of Embodiment>

The features and effects of the embodiment can be summarized as follows as an example.
(5-1) In the embodiment, a process (S103, S104) of generating the presentation screen 27 is executed by a computer (the system 1). The presentation screen 27 presents to a user U010 the classification content (for example, "T" or "N") of at least one label associated with at least one region image in a medical image by at least one of a plurality of annotations. Thereby, a useful judgment material can be presented by the presentation screen 27 at the time of an annotation work or the like. Labels by a plurality of annotations can be easily compared.

Here, for distinction, the user U010 may be referred to as a "first user", a user U001 may be referred to as a "second user", and a user U002 may be referred to as a "third user" for convenience.
(5-2) In the embodiment, as an example, the plurality of annotations may include a first annotation and a second annotation. The first annotation is performed by the first user U010. The second annotation may be one performed by the second user U001 who is different from the first user U010, or may be one performed by the first user U010 in before the first annotation. A presentation process (S104) may cause the computer (the system 1) to generate the presentation screen 27 so as to present to the first user U010 the classification content of at least one label associated with at least one region image in the medical image by the first and second annotations when the first user U010 is performing the first annotation or after the end of the first annotation. The presentation screen 27 may disclose information capable of identifying who the second user is, or may not disclose it.
(5-2-1) Specifically, as an example, when the first user U010 is performing the first annotation (S201-S202), a computer is caused to execute a process (S103, S104) of generating the presentation screen 27 so as to present to the first user U010 the classification content of a label associated with at least one region image in the medical image by the first annotation and the classification content of a label associated with at least one region image in the medical image by another second annotation (an annotation of the second user U001). Thereby, it is possible to compare the labels by each annotation between oneself (the user U010) who is the first user and the second user (the user U001 and/or U002) who is another person.
(5-2-2) As another example, when the first user U010 is performing a "second or subsequent annotation (U010-2)" (S201-S202), the computer (the system 1) may be caused to execute a process (S103, S104) of generating the presentation screen 27 so as to present to the first user U010 the classification content of a label associated with at least one region image in the medical image by this first annotation (U010-2) and the classification content of at least one label associated with at least one region image in the medical image by the second annotation (U010-1) performed in the past by the user U010. Thereby, it is possible to compare one of one's own annotations (U010-1) with another one (U010-2).

The "second annotation" is distinguishable from an arbitrary one annotation of the first user U010. A plurality of annotations (for example, U010-1, U010-2) saved with different annotation IDs by the same user (for example, the first user U010) may be handled as separate annotations. As an example, a case is considered where the user U010 is performing a second annotation (U010-2) on the same medical image. In this case, the "second annotation" may include one or more arbitrary annotations of another user (the second user U001 in the embodiment), and/or a past annotation (U010-1) of the first user U010.
(5-3) In the embodiment, as another example, the plurality of annotations may include a second annotation and a third annotation. When the first annotation is performed by the first user U010, the second and third annotations may be as follows. The second annotation may be one performed by the second user U001, or may be one performed by the first user U010 before the first annotation. The third annotation may be one performed by the third user U002, may be one performed by the first user U010 before the first annotation, or may be one performed by the second user U002 before the second annotation. The second and third annotations may be two annotations performed by the first user U010 before the first annotation, but it is assumed that the two annotations were performed at different points in time. The presentation process (S104) may cause the computer (the system 1) to generate the presentation screen 27 so as to present to the first user U010 the classification content of at least one label associated with at least one region image in the medical image by the second and third annotations when the first user U010 is performing the first annotation or after the end of the first annotation. The presentation screen 27 may disclose information capable of identifying who at least one of the second user and the third user is, or may not disclose it.
(5-3-1) Specifically, as an example, when the first user U010 is performing the first annotation, a process (S103, S104) of generating the presentation screen 27 so as to present to the user U010 the classification content of a label associated with at least one region image in the medical image by "each annotation of another plurality of users (the second user U001 and the third user U002)" may be executed by the computer (the system 1). It is possible to compare the labels by the annotations of a plurality of other persons (the second user U001 and the third user U002).
(5-3-2) Specifically, as another example, when the first user U010 is performing the first annotation, a process (S103, S104) of generating the presentation screen 27 so as to present to the user U010 the classification content of a label associated with at least one region image in the medical image by "each of the first and second annotations of the second user U001 (U001-1, U001-2) " may be executed by the computer (the system 1). It is possible to compare the labels by the second and third annotations (U001-1, U001-2) performed by the second user U001.

The presentation timing of the presentation screen 27 is not limited to during the first annotation work of the first user U010. The computer (the system 1) may be caused to present the presentation screen 27 when the first user U010 is not performing the first annotation (for example, after the end of the work). For example, a "reference mode" may be set on a menu screen after login, and in this reference mode, the computer (the system 1) may be caused to execute a process of generating the presentation screen 27 for the first user U010 to refer to his/her own and/or another's annotation without calling the work screen 26.

### <6. Others>

### (6-1. Criteria for substantial identity of images, and DB management)

In the embodiment, the following criteria of "substantial identity" can be adopted for the identity between a plurality of medical images. As an example of a case where two certain medical images are substantially identical, at least a first or a second situation below can be mentioned. In the first situation, the two medical images are completely identical and have the same image data. In the second situation, even if there is a difference between the medical images (for example, a difference in image data and/or an image ID), the difference can be regarded as identical for annotation purposes.

For example, it is assumed that a certain medical image is acquired by imaging the same medical image object. It is assumed that a new medical image is generated by duplicating the medical image data, changing an image format after duplication (e.g., saving in a compressed format), or applying an image processing (a change of resizing, brightness, contrast, saturation, color tone, etc.) after duplication. The original medical image and the new medical image are substantially identical even if there is a difference in the image ID, the image data, or the like. This is because an imaging object of the medical image is the same, and the difference can be ignored for annotation purposes. This is also the same for a region image, and the criteria of substantial identity can also be adopted for a region image generated from a substantially identical medical image.

Alternatively, for example, a mechanism called a "virtual slide" may be used. In the virtual slide, the same imaging object (a pathology specimen, that is, a tissue) is imaged at various magnifications, thereby acquiring, for example, about several to several tens of pathology images with different magnifications, and registering the numerous pathology images in the image DB. Since digitized pathology specimen data can be used, there is an advantage that an observation accuracy of a pathologist is easily improved. A group of pathology images in the virtual slide are substantially identical because they only represent the same pathology specimen at different magnifications. This concept is also the same for any medical image that images the same imaging object at different magnifications. The criteria of substantial identity can also be adopted for a region image generated from these substantially identical medical images.

Further, for example, when a tissue is continuously cut out to generate a continuous section, a certain pathology section and a pathology section located immediately above or below it may have almost the same structure. In that case, the pathology images of those respective pathology sections are substantially identical. A similar case is also assumed for any medical image other than a pathology image. For example, it is assumed that when a three-dimensional structure is sliced, planar images of adjacent layers are substantially identical. Also in these cases, the plurality of medical images are substantially identical. The criteria of substantial identity can also be adopted for a region image generated from these substantially identical medical images.

The embodiment can adopt at least the "criteria for substantial identity of images" described in each of the above examples. For example, in the embodiment, in the image DB of the storage unit 20, the image IDs may be linkable to each other by a user's manual operation or a computer's automatic processing so that a medical image group to be handled as substantially identical is handled together.

As an example, it is assumed that a first user (for example, U010) has performed an annotation on a first medical image. It is assumed that a second user and/or a third user (for example, U001 and/or U002) has performed an annotation on a second medical image. It is assumed that the second medical image is one obtained by duplicating the first medical image, or one obtained by duplicating the first medical image and applying image processing thereto. It is assumed that the first and second medical images are linked in the image DB, and both images are treated as identical. Even if FIGS. 7 to 18 and FIGS. 20 to 24 are referred to assuming such a case, it is understood that a plurality of labels are associated with each region image in the substantially identical first and second medical images by a plurality of annotations.

In the embodiment, when a plurality of labels (for example, a first label and a second label) are associated with each region image of a substantially identical medical image, there are several examples for ID management in each DB of the storage unit 20. In some examples, an annotation of a grid method may be performed on a plurality of substantially identical medical images, or a grid setting may be made in an annotation of the designated frame method. In this case, a grid of each medical image may be set with the same size and the same coordinates.

As a first example of ID management, a DB in the storage unit 20 may associate one region image ID with a label ID by each of a plurality of annotations. In this case, a group of region image IDs may be commonly adopted for a plurality of medical images. That is, simply, a region image ID associated with a certain label (the first label) may also be adopted as a region image ID associated with another label (the second label).

However, the present invention is not limited to this, and a second example of ID management may be as follows. There may be a case where one or more second medical images (copied medical images) are generated by copying a first medical image (a master medical image). Furthermore, image processing may be applied to the second medical image. At least in these cases, although the first and second medical images are substantially the same image, they have different image data, and different image IDs can be set. Therefore, as the second example of ID management, the image IDs of the first and second medical images may be linked in the image DB. In an image group consisting of a group of a master medical image ID and one or more copied medical image IDs, when a label ID is associated with one region image ID, the same label ID may be automatically associated with a medical image of the remaining image ID by following this association.

At least, an association among a medical image ID, a region image ID, and a label ID may be performed directly as in the first example or indirectly as in the second example. Thereby, a plurality of annotations may be performed on medical images that are substantially identical to each other, and a plurality of labels may be associable with one region image by a plurality of annotations.

### (6-2. Annotation by computer)

A subject that performs an annotation (an annotation subject) is not limited to a person and may include a computer. When a computer classifies an image by a rule-based classification process or by machine inference of a trained discrimination model, classification content (a classification result) is associated with each image, and this may be regarded as an annotation. A plurality of annotations for contrasting or comparing labels may have different subjects or the same subject. A user ID for a computer may be set in an account DB, and a unique annotation subject ID for each computer may be assigned at the time of registration in the label DB.

### (6-3. No limitation on hardware configuration)

In the example of FIG. 1, the system 1 is illustrated as one information processing apparatus, but the present embodiment is not limited to this. For example, the system 1 may be configured from one or a plurality of physical servers or the like, may be configured using a virtual server operating on a hypervisor, or may be configured using a cloud server. Further, processing of a program related to generation and display of the presentation screen 27 can be arbitrarily shared between the system 1 (the server 1) and a user terminal 3. It should be noted that the way of sharing the processing in a control flow of FIG. 4 is an example.

In the description and claims of the present application, "a computer" should be understood to mean "at least one computer" unless a different meaning is explicitly limited. "The computer" may include any one or more of a user terminal and/or a server. When "the computer" executes a certain program, all of the execution processing may be completed by a single computer, or a part of the execution processing may be executed by a first computer (for example, a server) and the rest of the processing may be executed by one or more other computers (for example, a user terminal or another server). The one or more other computers may be any one or more of second to n-th computers (n is an arbitrary integer of 3 or more). A generation process of the presentation screen may be executed by the user terminal. Alternatively, the generation process of the presentation screen may be executed by the server, and in this case, completed presentation screen data may be transmitted to the user terminal. For the generation process of the presentation screen, a part of the processing may be executed by the user terminal, and the remaining processing may be executed by the server. However, when it is specified that "a computer" should be interpreted with a meaning different from the above interpretation, the description is limited to that.

Although various embodiments according to the present invention have been described, these are presented as examples and are not intended to limit the scope of the invention. The novel embodiment can be implemented in various other forms, and various omissions, substitutions, and changes can be made without departing from the gist of the invention. The embodiment and its modifications are included in the scope and gist of the invention, and are included in the scope of the invention described in the claims and its equivalents.

### Reference Signs List

1: system (server), 2: communication network, 3: user terminal, 4: diagnostic device, 11: processor, 12: storage device, 13: communication IF, 20: storage unit, 21: display control unit, 22: input unit, 23: generation unit, 25: presentation unit, 25a: data acquisition unit, 25b: presentation screen generation unit, 25c: comparison unit, 25d: selection unit, 25d: presentation screen generation unit, 26: work screen, 27: presentation screen, 31: control unit, 32: storage unit, 33: communication unit, 34: operation input unit, 34: operation device, 34: display device, 35: speaker, 36: microphone, 37: camera, 38: operation device, M10: selection menu (type of medical image), M11: selection menu (designation of medical image), M12: selection menu (annotation disclosure setting), N11: display region, T10, V11: display frame, T110: mouse pointer, U010: user (first user), U001: user (second user), U002: user (third user), V12: presentation target frame, W10: display area, W11: overview display area

## Claims

1. A program for assisting in annotation of a medical image, wherein
the program causes a computer to execute a presentation process of generating a presentation screen, and
the presentation screen presents to a user classification content of at least one label associated with at least one region image in the medical image by at least one of a plurality of annotations.

2. The program according to claim 1, wherein
the plurality of annotations include a first annotation and a second annotation,
the first annotation is performed by a first user,
the second annotation is performed by a second user or is performed by the first user before the first annotation, and
the presentation process causes the computer to generate the presentation screen so as to present to the first user the classification content of the at least one label associated with the at least one region image by the first and second annotations, when the first user is performing the first annotation or after completion of the first annotation.

3. The program according to claim 1, wherein
the plurality of annotations include a second annotation and a third annotation,
when a first annotation is performed by a first user,
the second annotation is performed by a second user or is performed by the first user before the first annotation,
the third annotation is performed by a third user, is performed by the first user before the first annotation, or is performed by the second user before the second annotation, and
the presentation process causes the computer to generate the presentation screen so as to present to the first user the classification content of the at least one label associated with the at least one region image by the second and third annotations, when the first user is performing the first annotation or after completion of the first annotation.

4. The program according to any one of claims 1 to 3, wherein
the at least one region image is defined in the medical image by a grid or by a designated frame in each annotation.

5. The program according to any one of claims 1 to 3,
wherein the at least one region image includes a label-comparable region image,
the label-comparable region image is a region image with which a plurality of labels are associated by the plurality of annotations, and
the presentation process causes the computer to generate the presentation screen so as to represent the classification content of the plurality of labels associated with the label-comparable region image by the plurality of annotations in a manner that allows a user to visually compare them.

6. The program according to any one of claims 1 to 3, wherein
the at least one region image includes a label-comparable region image,
the label-comparable region image is a region image with which a plurality of labels are associated by the plurality of annotations,
the program causes the computer to execute a process of calculating a comparison result from comparing the classification content of the plurality of labels, and
the presentation screen presents the classification content of the plurality of labels associated with the label-comparable region image to a user by presenting the comparison result.

7. The program according to any one of claims 1 to 3, wherein
the presentation screen presents to a user the classification content of at least one label associated with a plurality of region images in the medical image by at least one of a plurality of annotations.

8. The program according to any one of claims 1 to 3, wherein
the presentation screen has a region image and a label image,
the region image is an image of the at least one region in the medical image,
the label image is an image corresponding to a label associated with the at least one region image, and
the program causes a computer to execute a process of varying a display mode of the region image and/or the label image on the presentation screen according to the classification content and/or according to a comparison result of a plurality of the labels when the plurality of labels are associated with the region image.

9. The program according to any one of claims 1 to 3, wherein
the classification content of the label includes an index value or is an index value, and
the index value represents how probable the classification content of the label is.

10. An assistance method in annotation of a medical image,
causing a computer to execute a presentation process of generating a presentation screen, wherein
the presentation screen presents to a user classification content of at least one label associated with at least one region image in the medical image by at least one of a plurality of annotations.

11. A system for assisting in annotation of a medical image,
comprising a presentation unit that generates a presentation screen, wherein
the presentation screen presents to a user classification content of at least one label associated with at least one region image in the medical image by at least one of a plurality of annotations.
